# EUROPEAN PATENT APPLICATION

(11) **EP 4 632 063 A2**
(43) Date of publication of application: **15.10.2025**
(21) Application number: 25183154.1
(22) Date of filing: 13.04.2021
(51) Int. Cl.: C12N 5/071

(54) **COPPER LOSS MITIGATION**

(30) Priority: 15.04.2020 US 202063010532 P
(62) Divisional of application: 21725853.2
(71) Applicant: GENENTECH, INC., South San Francisco, CA 94080 (US)
(72) Inventor: SHIRATORI, Masaru Ken, South San Francisco 94080 (US); KLAIR, Nathaniel Thomas, South San Francisco 94080 (US); BAKER, Jordan James, South San Francisco 94080 (US)
(74) Representative: HGF

(57) **Abstract**

Provided herein are methods of preventing loss of copper in a solution. The solution is a cell culture medium, cell culture feed or cell culture additive compromising copper(II) and cysteine. The method comprises having substantially the same amount of soluble copper before and after filtering the solution. Also provided are methods of preparing a cell culture medium, cell culture feed or cell culture additive comprising copper (II), cysteine, and other components, without loss of soluble copper. Also provided are methods of culturing a cell or making a biological product comprising use of media prepared according to said methods of preventing loss of copper or preparing said media. Further provided is media prepared according to said methods and use of such media.

## Description

### Cross Reference to Related Application

This application claims the benefit of priority to US Provisional Patent Application No. 63/010532 filed on 15 April 2020, the content of which is hereby incorporated by reference in its entirety.

This disclosure relates, in part, to a method of preventing copper loss in solution. Also provided are methods of preparing a cell culture medium, cell culture feed or cell culture additive comprising copper (II), cysteine, and other components, without loss of soluble copper. Cell culture medium, cell culture feed, or cell culture additive may be obtainable or obtained by these methods. Cells may be cultured in media prepared according to such methods and biological products may be made by said cells.

### BACKGROUND

Cell cultures may be used to create products such as recombinant proteins, or indeed the cultured cells themselves could be a product. Cell culture may use either eukaryotic cells, such as mammalian cells, or prokaryotic cells, such as bacterial cells.

Cell culture relies upon the use of medium to support and grow cells under controlled conditions. The medium and related feed, supplements or additives require the presence of many different components for successful cell culture. The development of chemically defined media (CDM) for animal cell culture that could support commercial production of recombinant proteins was a significant milestone for the biotechnology industry. CDM eliminated the variability inherent in serum/lysate containing media (Price PJ, Gregory EA. Relationship between in vitro growth promotion and biophysical and biochemical properties of the serum supplement, In Vitro Cellular & Developmental Biology-Plant, 1982;18(6), which is incorporated by reference herein in its entirety) and drastically reduced the risk of adventitious agents (van der Valk J, Mellor D, Brands R, et al., The humane collection of fetal bovine serum and possibilities for serum-free cell and tissue culture, Toxicology in Vitro. 2004;18(1):1-12, which is incorporated by reference herein in its entirety). Prior to CDM, serum or lysates were relied upon to deliver a range of nutrients and components necessary for cell growth.

CDM provides an opportunity to study media component interactions relative to undefined media (e.g., containing serum or hydrolysates). However, one advantage of serum/lysate containing media is that the compounds are typically already in bioavailable forms. For example, iron, while an essential cofactor for many cellular functions, can be toxic to cells. In undefined media, biological chelators such as transferrin are relied upon to deliver iron without toxicity (Bjare U. Serum-free cell culture. Pharmacology & Therapeutics. 1992;53(3):355-374, which is incorporated by reference herein in its entirety). In CDM, non-protein chelators such as citrate must be studied and implemented to prevent iron toxicity. CDM typically contain a large number of components, which can interact in various manners that not necessarily readily apparent. Successful implementation of CDM requires understanding how the components chemically interact and how to keep them in biologically available forms. Understanding about how the components interact is therefore also important in obtaining optimal media and supplements that comprise undefined media.

There is accordingly a need for improved methods of preparing cell culture media, feeds and supplements, to reduce potential issues due to interactions between components.

### SUMMARY OF THE DISCLOSURE

We have identified that in cell culture media, feeds, or additives comprising cysteine, cysteine oxidation to cystine readily occurs in the presence of trace metals catalysts, primarily copper and iron. When copper catalyzes cysteine oxidation it is converted to an insoluble copper (I) form. Once there is no cysteine left to be oxidized, the copper usually returns to a soluble form. If, however, the solution goes through sterile filtration while cysteine oxidation is occurring, at least a proportion of the copper will typically be in an insoluble form and will be removed from the media, reducing the level of copper in the medium. This is undesirable, as copper is a trace metal that is required in many cell cultures, so the loss of copper from the solution means that the resulting media will provide less copper to any cultured cells that what was intended. This will typically result in lower productivity. An object of the present disclosure is therefore to minimize and/or avoid loss of copper from solutions (such as cell culture medium, cell culture feed or cell culture additive) during filtration.

In a first aspect the present disclosure provides a method of preventing loss of copper in a solution. The solution is a cell culture medium, cell culture feed or cell culture additive comprising copper (II) and cysteine. The method comprises having substantially the same amount of soluble copper before and after filtering the solution.

In an embodiment, the filtering is performed with the solution comprising a dissolved oxygen (dO₂) at at least a specified level. Maintaining the level of oxygen at at least a specified level may rapidly convert copper (I) into a soluble copper (II), preventing the accumulation of appreciable amounts of insoluble copper (I). The specified level may be a level of at least about 6% dO₂. The specified level may be a level of at least about 8% dO₂, e.g. at a level of at least about 10% dO₂ or at a level of at least about 12% dO₂. The filtering may be performed with the solution comprising a dissolved oxygen (dO₂) level of at least about 6% dO₂. The method may further comprise maintaining the dO₂ level of the solution at at least about 8% dO₂ (e.g. at a level of at least about 10% dO₂ or at a level of at least about 12% dO₂) for at least about 10 minutes prior to commencing the filtering. The method may further comprise maintaining the dO₂ level of the solution at at least about 6% dO₂ for at least about 10 minutes prior to commencing the filtering. The method may further comprise maintaining the dO₂ level of the solution at at least about 8% dO₂ (e.g. at a level of at least about 10% dO₂ or at a level of at least about 12% dO₂) for at least about 15 minutes prior to commencing the filtering. The method may further comprise maintaining the dO₂ level of the solution at at least about 6% dO₂ for at least about 15 minutes prior to commencing the filtering.

The specified level may be a level of at least about 0.4 mg/L dO₂, e.g. at a level of at least about 0.6 mg/L dO₂ or at a level of at least about 0.8 mg/L dO₂. The filtering may be performed with the solution comprising a dissolved oxygen (dO₂) level of at least about 0.3 mg/L dO₂. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 10 minutes prior to commencing the filtering. The method may further comprise maintaining the dO₂ level of the solution at at least about 0.3 mg/L dO₂ for at least about 10 minutes prior to commencing the filtering. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 15 minutes prior to commencing the filtering. The method may further comprise maintaining the dO₂ level of the solution at at least about 0.3 mg/L dO₂ for at least about 15 minutes prior to commencing the filtering.

In an embodiment, the dO₂ levels are maintained at at least the required level by oxygenating the solution and / or optimizing the filtering. The required level may be about 6% dO₂, about 8% dO₂, about 10% dO₂ or about 12% dO₂. The required level may be about 8% dO₂. The required level may be about 0.3 mg/L dO₂, about 0.4 mg/L dO₂, about 0.6 mg/L dO₂, or about 0.8 mg/L dO₂. The required level may be about 0.4 mg/L dO₂.

The oxygenating may comprise sparging the solution with a gas comprising oxygen. The oxygenating may comprise agitating and/or mixing the solution to increase contact of the solution with gas comprising oxygen. The oxygenating may comprise sparging the solution with a gas comprising oxygen and agitating or mixing the solution to increase contact of the solution with gas comprising oxygen. The gas comprising oxygen may be atmosphere.

Optimizing the filtering may comprise increasing filtration rate, and/or filter size, and/or filter capacity, such that the dO₂ levels do not fall below a specified level during filtering. The specified level may be about 6% dO₂, about 8% dO₂, about 10% dO₂ or about 12% dO₂. The specified level may be about 8% dO₂. The specified level may be about 0.3 mg/L dO₂, about 0.4 mg/L dO₂, about 0.6 mg/L dO₂, or about 0.8 mg/L dO₂. The specified level may be about 0.4 mg/L dO₂.

The increasing may comprise increasing the filtration rate (and optionally the filter size, and/or optionally the filter capacity) by at least about 10%. The increasing may comprise increasing the filtration rate (and optionally the filter size, and/or optionally the filter capacity) by at least about 15%. The increasing may comprise increasing the filtration rate (and optionally the filter size, and/or optionally the filter capacity) by at least about 20%. The increasing may comprise increasing the filtration rate (and optionally the filter size, and/or optionally the filter capacity) by at least about 25%. The increasing may comprise increasing the filtration rate (and optionally the filter size, and/or optionally the filter capacity) by at least about 30%. The increasing may comprise increasing the filtration rate (and optionally the filter size, and/or optionally the filter capacity) by at least about 50%. The increasing may comprise increasing the filtration rate (and optionally the filter size, and/or optionally the filter capacity) by at least about 75%. The increasing may comprise increasing the filtration rate (and optionally the filter size, and/or optionally the filter capacity) by at least about 100%. The increasing may comprise increasing the filter size (and optionally the filter capacity) by at least about 10%. The increasing may comprise increasing the filter size (and optionally the filter capacity) by at least about 15%. The increasing may comprise increasing the filter size (and optionally the filter capacity) by at least about 20%. The increasing may comprise increasing the filter size (and optionally the filter capacity) by at least about 25%. The increasing may comprise increasing the filter size (and optionally the filter capacity) by at least about 30%. The increasing may comprise increasing the filter size (and optionally the filter capacity) by at least about 50%. The increasing may comprise increasing the filter size (and optionally the filter capacity) by at least about 75%. The increasing may comprise increasing the filter size (and optionally the filter capacity) by at least about 100%. The increasing may comprise increasing the filter capacity by at least about 10%. The increasing may comprise increasing the filter capacity by at least about 15%. The increasing may comprise increasing the filter capacity by at least about 20%. The increasing may comprise increasing the filter capacity by at least about 25%. The increasing may comprise increasing the filter capacity by at least about 30%. The increasing may comprise increasing the filter capacity by at least about 50%. The increasing may comprise increasing the filter capacity by at least about 75%. The increasing may comprise increasing the filter capacity by at least about 100%.

The filtering may be completed within about 24 hours of adding copper (II) to the solution. The filtering may be completed within about 18 hours of adding copper (II) to the solution. The filtering may be completed within about 12 hours of adding copper (II) to the solution. The filtering may be completed within about 9 hours of adding copper (II) to the solution. The filtering may be completed within about 6 hours of adding copper (II) to the solution. The filtering may be completed within about 5 hours of adding copper (II) to the solution. The filtering may be completed within about 4 hours of adding copper (II) to the solution. The filtering may be completed within about 3 hours of adding copper (II) to the solution. The filtering may be completed within about 2 hours of adding copper (II) to the solution. The filtering may be completed within about 1 hour of adding copper (II) to the solution. The filtering may be completed within about 30 minutes of adding copper (II) to the solution. The filtering may be completed within about 20 minutes of adding copper (II) to the solution. The filtering may be completed within about 15 minutes of adding copper (II) to the solution. The filtering may be completed within about 10 minutes of adding copper (II) to the solution.

The filtering may be performed on a volume of not more that about 100,000 L. The filtering may be performed on a volume of not more than about 50,000 L. The filtering may be performed on a volume of not more than about 25,000 L. The filtering may be performed on a volume of not more than about 25,000 L. The filtering may be performed on a volume of not more than about 15,000 L. The filtering may be performed on a volume of not more than about 10,000 L. The filtering may be performed on a volume of not more than about 5,000 L. The filtering may be performed on a volume of not more than about 4,000 L. The filtering may be performed on a volume of not more than about 3,000 L. The filtering may be performed on a volume of not more than about 2,000 L. The filtering may be performed on a volume of not more than about 1,000 L. The filtering may be performed on a volume of not more than about 500 L. The filtering may be performed on a volume of not more than about 250 L. The filtering may be performed on a volume of not more than about 100 L.

The filtering may be performed on a volume of at least about 50 L. The filtering may be performed at a volume of at least about 100 L. For example, the filtering may be performed on a volume of about 100 L to about 2,000 L, or the filtering may be performed at a volume of about 100 L to about 25,000 L. The filtering may be performed on a volume of at least 1,000 L. For example, the filtering may be performed on a volume of about 1,000 L to about 2,000 L, or the filtering may be performed at a volume of about 1,000 L to about 25,000 L.

The filtering may be completed within about 18 hours (e.g. within about 12 hours, within about 9 hours, or within about 6 hours) of adding copper (II) to the solution comprising a volume of not more than about 25,000 L. The filtering may be completed within about 12 hours (e.g. within about 9 hours, within about 6 hours, or within about 3 hours) of adding copper (II) to the solution comprising a volume of not more than about 15,000 L. The filtering may be completed within about 9 hours (e.g. within about 6 hours, within about 3 hours, or within about 1 hour) of adding copper (II) to the solution comprising a volume of not more than about 10,000 L. The filtering may be completed within about 9 hours (e.g. within about 6 hours, within about 3 hours, or within about 1 hour) of adding copper (II) to the solution comprising a volume of not more than about 5,000 L. The filtering may be completed within about 1 hour (e.g. within about 30 minutes or within about 20 minutes) of adding copper (II) to the solution comprising a volume of not more than about 2,000 L. The filtering may be completed within about 30 minutes (e.g. within about 20 minutes or within about 10 minutes) of adding copper (II) to the solution comprising a volume of not more than about 1,000 L.

The filtering may be performed at a rate of at least about 5 L/min, 10 L/min, about 20 L/min, about 30 L/min, about 40 L/min, about 50 L/min, about 60 L/min, about 70 L/min, or about 80 L/min. The filtering may be performed at a rate of at least 10 L/min. The filtering may be performed at a rate of at least 20 L/min. The filtering may be performed at a rate of at least 30 L/min. The filtering may be performed at a rate of at least 40 L/min. The filtering may be performed at a rate of at least 50 L/min.

The filtering may be performed at a rate of not more than about 170 L/min, about 150 L/min, about 130 L/min, about 110 L/min, about 100 L/min, about 90 L/min, or about 80 L/min. The filtering may be performed at a rate of not more than about 170 L/min. The filtering may be performed at a rate of not more than about 150 L/min. The filtering may be performed at a rate of not more than about 130 L/min. The filtering may be performed at a rate of not more than about 110 L/min. The filtering may be performed at a rate of not more than about 100 L/min.

The filtering may be performed at a rate of from about 5 L/min to about 170 L/min. The filtering may be performed at a rate of from about 10 L/min to about 150 L/min. The filtering may be performed at a rate of from about 20 L/min to about 150 L/min. The filtering may be performed at a rate of from about 30 L/min to about 150 L/min. The filtering may be performed at a rate of from about 40 L/min to about 150 L/min. The filtering may be performed at a rate of from about 50 L/min to about 150 L/min. The filtering may be performed at a rate of from about 10 L/min to about 130 L/min. The filtering may be performed at a rate of from about 20 L/min to about 130 L/min. The filtering may be performed at a rate of from about 30 L/min to about 130 L/min. The filtering may be performed at a rate of from about 40 L/min to about 130 L/min. The filtering may be performed at a rate of from about 50 L/min to about 130 L/min. The filtering may be performed at a rate of from about 10 L/min to about 100 L/min. The filtering may be performed at a rate of from about 20 L/min to about 100 L/min. The filtering may be performed at a rate of from about 30 L/min to about 100 L/min. The filtering may be performed at a rate of from about 40 L/min to about 100 L/min. The filtering may be performed at a rate of from about 50 L/min to about 100 L/min.

The filtering may be performed at a rate of at least 0.1% of the total volume per minute. The filtering may be performed at a rate of at least 0.25% of the total volume per minute. The filtering may be performed at a rate of at least 0.5% of the total volume per minute. The filtering may be performed at a rate of at least 1% of the total volume per minute. The filtering may be performed at a rate of at least 2.5% of the total volume per minute. The filtering may be performed at a rate of at least 5% of the total volume per minute.

The method may comprise monitoring the level of oxygen prior to commencing the filtering, for example monitoring the level of oxygen for at least about 10 minutes (e.g. for at least about 15 minutes) prior to commencing the filtering. The method may comprise monitoring the level of dO₂ during filtering. The method may comprise monitoring the level of oxygen prior to commencing the filtering and monitoring the level of dO₂ during filtering. When the method comprises monitoring the level of oxygen, maintaining the level of oxygen at at least the specified level may comprise adding oxygen to the solution in response to the monitored level of oxygen approaching the specified level. When the method comprises monitoring the level of oxygen, maintaining the level of oxygen at at least the specified level may comprise increasing oxygenating the solution and/or optimizing the filtering in response to the monitored level of oxygen approaching the specified level. The level of dO₂ may be monitored using any suitable technique. The level of dO₂ may be monitored with an electrochemical oxygen sensor or an optical oxygen sensor. The level of dO₂ may be monitored with an electrochemical oxygen sensor. The level of dO₂ may be monitored with an optical oxygen sensor.

In embodiments, the method comprises providing a cysteine solution and a copper (II) solution; and the filtering comprises separately filtering the cysteine solution and the copper (II) solution, and then combining the filtered cysteine solution and the filtered copper (II) solution to form the cell culture medium, cell culture feed or cell culture additive. By keeping the copper (II) solution and cysteine solution separate until after filtering, the copper is retained in soluble copper (II) before and during filtering.

The cysteine solution may comprise at least the majority of the components of the cell culture medium, cell culture feed or cell culture additive, other than copper. The cysteine solution may comprise substantially all of the components of the cell culture medium, cell culture feed or cell culture additive other than copper and other trace elements such as iron. The cysteine solution may comprise substantially all of the components of the cell culture medium, cell culture feed or cell culture additive other than copper and iron. The cysteine solution may comprise substantially all of the components of the cell culture medium, cell culture feed or cell culture additive other than copper.

The copper solution may comprise at least the majority of the components of the cell culture medium, cell culture feed or cell culture additive, other than cysteine.

The concentration of the copper in the copper (II) solution may be in the range of about of about 0.005 µM to about 1.5 M, for example 0.005 µM to about 1 M. The upper limit of this range may be temperature dependent. Thus solutions that are intended for use at a temperature of at least about 30 °C may have a concentration of up to about 1.5 M, while compounds intended for use or storage at less than room temperature (such as a temperature of less than about 20 °C, e.g. at a temperature of about 10 °C) may have a concentration of up to about 1 M. Where the final solution is a cell culture medium, the concentration of copper in the copper (II) solution may be in the range of about 0.005 µM to about 5 µM. Where the final solution is a cell culture feed, the concentration of copper in the copper (II) solution may be in the range of about 0.1 mM to about 150 mM. Where the final solution is a cell culture additive, the concentration of copper in the copper (II) solution may be in the range of about 1 mM to about 1.5 M, e.g. about 1 mM to about 1 M.

In embodiments, the filtering comprises sterile filtering. The sterile filtering may comprise use of a filtration medium having a pore size of not more than about 0.5 µm. The sterile filtering may comprise use of a filtration medium having a pore size of not more than about 0.2 µm. The sterile filtering may comprise use of a filtration medium having a pore size of not more than about 0.1 µm. The sterile filtering may comprise use of a filtration medium having a pore size of not more than about 0.1 µm. In embodiments, the filtration medium comprises one or more membranes, hollow fibers, columns, spiral membranes, tubes, capillaries, capsules, cassettes, discs, frits, or plugs.

In embodiments, the cysteine is provided substantially as a cysteine derivative that does not comprise a sulfhydryl group. For example, the cysteine may comprise less than about 1 mM free cysteine, e.g. the cysteine may comprise less than 0.5 mM free cysteine (or less than 0.1 mM free cysteine). Cysteine derivatives that do not comprise sulfhydryl groups do not react with copper (II) to form insoluble copper (I).

The cysteine may be converted substantially into a cysteine derivative that does not comprise a sulfhydryl group prior to adding the copper (II) to the solution. The cysteine derivative may comprise a disulfide and/or a thiazolidine moiety.

The cysteine derivative may comprises a disulfide. For example, the cysteine derivative may comprise cystine, S-sulfocysteine, S-sulfocysteinylglycine, L-cysteine mixed disulfides, and/or S-sulfoglutathione. The cysteine derivative may comprise cystine.

The cysteine derivative may comprise a thiazolidine moiety. For example, the cysteine derivative may comprise 4-carboxy-2-methylthiazolidine-2-carboxylate, 2-methyl-1,3-thiazolidine-2,4-dicarboxylic acid, 2-(2-carboxyethyl)21,3-thiazolidine-2,4-dicarboxylic acid, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine). The cysteine derivative may comprise 4-carboxy-2-methylthiazolidine-2-carboxylate.

The cysteine derivative may comprise at least one of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g. FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid, or carbohydrate based thiazolidines (e.g. D-ribose-L-cysteine).

In embodiments, the copper may be in the form of a copper (II) chelate. The chelate may comprise at least one chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2).

In a second aspect, the disclosure provides a method of preparing a cell culture medium, cell culture feed or cell culture additive comprising copper (II), cysteine, and other components, without loss of soluble copper. The method comprises providing an aqueous solution comprising cysteine and other components; providing an aqueous solution comprising copper (II); separately filtering the aqueous solution comprising cysteine and other components, and the aqueous solution comprising copper (II); and combining the filtered solutions to provide the cell culture medium, cell culture feed or cell culture additive.

Preparation of the aqueous solution comprising cysteine and other components may comprise dissolving and/or solubilizing cysteine and other components in an aqueous solvent system (e.g. water). Preparation of the solution comprising cysteine and other components may comprise dissolving and/or solubilizing cysteine and other components in an initial solvent system comprising acid, base, or water miscible organic solvent, followed by dilution of the initial solvent system with water and optionally other components to form the aqueous solution.

Preparation of the aqueous solution comprising copper (II) may comprise dissolving a copper (II) salt in an aqueous solvent system (e.g. water). Preparation of the solution comprising copper (II) may comprise dissolving and/or solubilizing a copper (II) salt in an initial solvent system comprising acid, base, or water miscible organic solvent, followed by dilution of the initial solvent system with water and optionally other components to form the aqueous solution.

The acid may be an inorganic or organic acid. Exemplary inorganic acids include hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like. Exemplary organic acids include relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Exemplary bases include ammonia; hydroxides of sodium, potassium, calcium, ammonium, organic amino, or magnesium and the like; carbonates or bicarbonates of sodium, potassium, or ammonium. Exemplary water miscible organic solvents include ethanol, methanol, acetone, dimethylsulfoxide (DMSO) and dimethylformadine.

The concentration of the copper in the copper (II) solution may be in the range of about of about 0.005 µM to about 1 M. Where the final solution is a cell culture medium, the concentration of copper in the copper (II) solution may be in the range of about 0.005 µM to about 5 µM. Where the final solution is a cell culture feed, the concentration of copper in the copper (II) solution may be in the range of about 0.1 mM to about 150 mM. Where the final solution is a cell culture additive, the concentration of copper in the copper (II) solution may be in the range of about 1 mM to about 1 M.

In an embodiment, the filtering is performed with the solution comprising a dissolved oxygen (dO₂) at at least a specified level. Maintaining the level of oxygen at at least a specified level may rapidly convert copper (I) into a soluble copper (II), preventing the accumulation of appreciable amounts of insoluble copper (I). The specified level may be a level of at least about 6% dO₂. The specified level may be a level of at least about 8% dO₂, e.g. at a level of at least about 10% dO₂ or at a level of at least about 12% dO₂. The filtering may be performed with the solution comprising a dissolved oxygen (dO₂) level of at least about 6% dO₂. The method may further comprise maintaining the dO₂ level of the solution at at least about 8% dO₂ (e.g. at a level of at least about 10% dO₂ or at a level of at least about 12% dO₂) for at least about 10 minutes prior to commencing the filtering. The method may further comprise maintaining the dO₂ level of the solution at at least about 6% dO₂ for at least about 10 minutes prior to commencing the filtering. The method may further comprise maintaining the dO₂ level of the solution at at least about 8% dO₂ (e.g. at a level of at least about 10% dO₂ or at a level of at least about 12% dO₂) for at least about 15 minutes prior to commencing the filtering. The method may further comprise maintaining the dO₂ level of the solution at at least about 6% dO₂ for at least about 15 minutes prior to commencing the filtering.

The specified level may be a level of at least about 0.4 mg/L dO₂, e.g. at a level of at least about 0.6 mg/L dO₂ or at a level of at least about 0.8 mg/L dO₂. The filtering may be performed with the solution comprising a dissolved oxygen (dO₂) level of at least about 0.3 mg/L dO₂. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 10 minutes prior to commencing the filtering. The method may further comprise maintaining the dO₂ level of the solution at at least about 0.3 mg/L dO₂ for at least about 10 minutes prior to commencing the filtering. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 15 minutes prior to commencing the filtering. The method may further comprise maintaining the dO₂ level of the solution at at least about 0.3 mg/L dO₂ for at least about 15 minutes prior to commencing the filtering.

In an embodiment, the dO₂ levels are maintained at at least the required level by oxygenating the solution and / or optimizing the filtering. The required level may be about 6% dO₂, about 8% dO₂, about 10% dO₂ or about 12% dO₂. The required level may be about 8% dO₂. The required level may be about 0.3 mg/L dO₂, about 0.4 mg/L dO₂, about 0.6 mg/L dO₂, or about 0.8 mg/L dO₂. The required level may be about 0.4 mg/L dO₂.

The oxygenating may comprise sparging the solution with a gas comprising oxygen. The oxygenating may comprise agitating and/or mixing the solution to increase contact of the solution with gas comprising oxygen. The oxygenating may comprise sparging the solution with a gas comprising oxygen and agitating or mixing the solution to increase contact of the solution with gas comprising oxygen. The gas comprising oxygen may be atmosphere.

Optimizing the filtering may comprise increasing filtration rate, and/or filter size, and/or filter capacity, such that the dO₂ levels do not fall below a specified level during filtering. The specified level may be about 6% dO₂, about 8% dO₂, about 10% dO₂ or about 12% dO₂. The specified level may be about 8% dO₂. The specified level may be about 0.3 mg/L dO₂, about 0.4 mg/L dO₂, about 0.6 mg/L dO₂, or about 0.8 mg/L dO₂. The specified level may be about 0.4 mg/L dO₂.

The increasing may comprise increasing the filtration rate (and optionally the filter size, and/or optionally the filter capacity) by at least about 10%. The increasing may comprise increasing the filtration rate (and optionally the filter size, and/or optionally the filter capacity) by at least about 15%. The increasing may comprise increasing the filtration rate (and optionally the filter size, and/or optionally the filter capacity) by at least about 20%. The increasing may comprise increasing the filtration rate (and optionally the filter size, and/or optionally the filter capacity) by at least about 25%. The increasing may comprise increasing the filtration rate (and optionally the filter size, and/or optionally the filter capacity) by at least about 30%. The increasing may comprise increasing the filtration rate (and optionally the filter size, and/or optionally the filter capacity) by at least about 50%. The increasing may comprise increasing the filtration rate (and optionally the filter size, and/or optionally the filter capacity) by at least about 75%. The increasing may comprise increasing the filtration rate (and optionally the filter size, and/or optionally the filter capacity) by at least about 100%. The increasing may comprise increasing the filter size (and optionally the filter capacity) by at least about 10%. The increasing may comprise increasing the filter size (and optionally the filter capacity) by at least about 15%. The increasing may comprise increasing the filter size (and optionally the filter capacity) by at least about 20%. The increasing may comprise increasing the filter size (and optionally the filter capacity) by at least about 25%. The increasing may comprise increasing the filter size (and optionally the filter capacity) by at least about 30%. The increasing may comprise increasing the filter size (and optionally the filter capacity) by at least about 50%. The increasing may comprise increasing the filter size (and optionally the filter capacity) by at least about 75%. The increasing may comprise increasing the filter size (and optionally the filter capacity) by at least about 100%. The increasing may comprise increasing the filter capacity by at least about 10%. The increasing may comprise increasing the filter capacity by at least about 15%. The increasing may comprise increasing the filter capacity by at least about 20%. The increasing may comprise increasing the filter capacity by at least about 25%. The increasing may comprise increasing the filter capacity by at least about 30%. The increasing may comprise increasing the filter capacity by at least about 50%. The increasing may comprise increasing the filter capacity by at least about 75%. The increasing may comprise increasing the filter capacity by at least about 100%.

The method may comprise monitoring the level of oxygen prior to commencing the filtering, for example monitoring the level of oxygen for at least about 10 minutes (e.g. for at least about 15 minutes) prior to commencing the filtering. The method may comprise monitoring the level of dO₂ during filtering. The method may comprise monitoring the level of oxygen prior to commencing the filtering and monitoring the level of dO₂ during filtering. When the method comprises monitoring the level of oxygen, maintaining the level of oxygen at at least the specified level may comprise adding oxygen to the solution in response to the monitored level of oxygen approaching the specified level. When the method comprises monitoring the level of oxygen, maintaining the level of oxygen at at least the specified level may comprise increasing oxygenating the solution and/or optimizing the filtering in response to the monitored level of oxygen approaching the specified level. The level of dO₂ may be monitored using any suitable technique. The level of dO₂ may be monitored with an electrochemical oxygen sensor or an optical oxygen sensor. The level of dO₂ may be monitored with an electrochemical oxygen sensor. The level of dO₂ may be monitored with an optical oxygen sensor.

In embodiments, the cysteine is provided substantially as a cysteine derivative that does not comprise a sulfhydryl group. For example, the cysteine may comprise less than about 1 mM free cysteine, e.g. the cysteine may comprise less than 0.5 mM free cysteine (or less than 0.1 mM free cysteine). Cysteine derivatives that do not comprise sulfhydryl groups do not react with copper (II) to form insoluble copper (I).

The cysteine may be converted substantially into a cysteine derivative that does not comprise a sulfhydryl group prior to adding the copper (II) to the solution. The cysteine derivative may comprise a disulfide and/or a thiazolidine moiety.

The cysteine derivative may comprises a disulfide. For example, the cysteine derivative may comprise cystine, S-sulfocysteine, S-sulfocysteinylglycine, L-cysteine mixed disulfides, and/or S-sulfoglutathione. The cysteine derivative may comprise cystine.

The cysteine derivative may comprise a thiazolidine moiety. For example, the cysteine derivative may comprise 4-carboxy-2-methylthiazolidine-2-carboxylate, 2-methyl-1,3-thiazolidine-2,4-dicarboxylic acid, 2-(2-carboxyethyl)21,3-thiazolidine-2,4-dicarboxylic acid, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine). The cysteine derivative may comprise 4-carboxy-2-methylthiazolidine-2-carboxylate.

The cysteine derivative may comprise at least one of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g. FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid, or carbohydrate based thiazolidines (e.g. D-ribose-L-cysteine).

In embodiments, the copper may be in the form of a copper (II) chelate. The chelate may comprise at least one chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2).

In third aspect, the disclosure provides a method of preparing a cell culture medium, cell culture feed or cell culture additive comprising copper (II), cysteine, and other components, without loss of soluble copper. The method comprises filtering a solution comprising the copper (II), cysteine, and other components to provide the cell culture medium, cell culture feed or cell culture additive.

The filtering may be completed within about 24 hours of adding copper (II) to the solution. The filtering may be completed within about 18 hours of adding copper (II) to the solution. The filtering may be completed within about 12 hours of adding copper (II) to the solution. The filtering may be completed within about 9 hours of adding copper (II) to the solution. The filtering may be completed within about 6 hours of adding copper (II) to the solution. The filtering may be completed within about 5 hours of adding copper (II) to the solution. The filtering may be completed within about 4 hours of adding copper (II) to the solution. The filtering may be completed within about 3 hours of adding copper (II) to the solution. The filtering may be completed within about 2 hours of adding copper (II) to the solution. The filtering may be completed within about 1 hour of adding copper (II) to the solution. The filtering may be completed within about 30 minutes of adding copper (II) to the solution. The filtering may be completed within about 20 minutes of adding copper (II) to the solution. The filtering may be completed within about 15 minutes of adding copper (II) to the solution. The filtering may be completed within about 10 minutes of adding copper (II) to the solution.

The filtering may be performed on a volume of not more that about 100,000 L. The filtering may be performed on a volume of not more than about 50,000 L. The filtering may be performed on a volume of not more than about 25,000 L. The filtering may be performed on a volume of not more than about 25,000 L. The filtering may be performed on a volume of not more than about 15,000 L. The filtering may be performed on a volume of not more than about 10,000 L. The filtering may be performed on a volume of not more than about 5,000 L. The filtering may be performed on a volume of not more than about 4,000 L. The filtering may be performed on a volume of not more than about 3,000 L. The filtering may be performed on a volume of not more than about 2,000 L. The filtering may be performed on a volume of not more than about 1,000 L. The filtering may be performed on a volume of not more than about 500 L. The filtering may be performed on a volume of not more than about 250 L. The filtering may be performed on a volume of not more than about 100 L.

The filtering may be performed on a volume of at least about 50 L. The filtering may be performed at a volume of at least about 100 L. For example, the filtering may be performed on a volume of about 100 L to about 2,000 L, or the filtering may be performed at a volume of about 100 L to about 25,000 L. The filtering may be performed on a volume of at least 1,000 L. For example, the filtering may be performed on a volume of about 1,000 L to about 2,000 L, or the filtering may be performed at a volume of about 1,000 L to about 25,000 L.

The filtering may be completed within about 18 hours (e.g. within about 12 hours, within about 9 hours, or within about 6 hours) of adding copper (II) to the solution comprising a volume of not more than about 25,000 L. The filtering may be completed within about 12 hours (e.g. within about 9 hours, within about 6 hours, or within about 3 hours) of adding copper (II) to the solution comprising a volume of not more than about 15,000 L. The filtering may be completed within about 9 hours (e.g. within about 6 hours, within about 3 hours, or within about 1 hour) of adding copper (II) to the solution comprising a volume of not more than about 10,000 L. The filtering may be completed within about 9 hours (e.g. within about 6 hours, within about 3 hours, or within about 1 hour) of adding copper (II) to the solution comprising a volume of not more than about 5,000 L. The filtering may be completed within about 1 hour (e.g. within about 30 minutes or within about 20 minutes) of adding copper (II) to the solution comprising a volume of not more than about 2,000 L. The filtering may be completed within about 30 minutes (e.g. within about 20 minutes or within about 10 minutes) of adding copper (II) to the solution comprising a volume of not more than about 1,000 L.

The filtering may be performed at a rate of at least about 5 L/min, 10 L/min, about 20 L/min, about 30 L/min, about 40 L/min, about 50 L/min, about 60 L/min, about 70 L/min, or about 80 L/min. The filtering may be performed at a rate of at least 10 L/min. The filtering may be performed at a rate of at least 20 L/min. The filtering may be performed at a rate of at least 30 L/min. The filtering may be performed at a rate of at least 40 L/min. The filtering may be performed at a rate of at least 50 L/min.

The filtering may be performed at a rate of not more than about 170 L/min, about 150 L/min, about 130 L/min, about 110 L/min, about 100 L/min, about 90 L/min, or about 80 L/min. The filtering may be performed at a rate of not more than about 170 L/min. The filtering may be performed at a rate of not more than about 150 L/min. The filtering may be performed at a rate of not more than about 130 L/min. The filtering may be performed at a rate of not more than about 110 L/min. The filtering may be performed at a rate of not more than about 100 L/min.

The filtering may be performed at a rate of from about 5 L/min to about 170 L/min. The filtering may be performed at a rate of from about 10 L/min to about 150 L/min. The filtering may be performed at a rate of from about 20 L/min to about 150 L/min. The filtering may be performed at a rate of from about 30 L/min to about 150 L/min. The filtering may be performed at a rate of from about 40 L/min to about 150 L/min. The filtering may be performed at a rate of from about 50 L/min to about 150 L/min. The filtering may be performed at a rate of from about 10 L/min to about 130 L/min. The filtering may be performed at a rate of from about 20 L/min to about 130 L/min. The filtering may be performed at a rate of from about 30 L/min to about 130 L/min. The filtering may be performed at a rate of from about 40 L/min to about 130 L/min. The filtering may be performed at a rate of from about 50 L/min to about 130 L/min. The filtering may be performed at a rate of from about 10 L/min to about 100 L/min. The filtering may be performed at a rate of from about 20 L/min to about 100 L/min. The filtering may be performed at a rate of from about 30 L/min to about 100 L/min. The filtering may be performed at a rate of from about 40 L/min to about 100 L/min. The filtering may be performed at a rate of from about 50 L/min to about 100 L/min.

The filtering may be performed at a rate of at least 0.1% of the total volume per minute. The filtering may be performed at a rate of at least 0.25% of the total volume per minute. The filtering may be performed at a rate of at least 0.5% of the total volume per minute. The filtering may be performed at a rate of at least 1% of the total volume per minute. The filtering may be performed at a rate of at least 2.5% of the total volume per minute. The filtering may be performed at a rate of at least 5% of the total volume per minute.

In embodiments, the filtering comprises sterile filtering. The sterile filtering may comprise use of a filtration medium having a pore size of not more than about 0.5 µm. The sterile filtering may comprise use of a filtration medium having a pore size of not more than about 0.2 µm. The sterile filtering may comprise use of a filtration medium having a pore size of not more than about 0.1 µm. The sterile filtering may comprise use of a filtration medium having a pore size of not more than about 0.1 µm.

In an embodiment, the filtering is performed with the solution comprising a dissolved oxygen (dO₂) at at least a specified level. Maintaining the level of oxygen at at least a specified level may rapidly convert copper (I) into a soluble copper (II), preventing the accumulation of appreciable amounts of insoluble copper (I). The specified level may be a level of at least about 6% dO₂. The specified level may be a level of at least about 8% dO₂, e.g. at a level of at least about 10% dO₂ or at a level of at least about 12% dO₂. The filtering may be performed with the solution comprising a dissolved oxygen (dO₂) level of at least about 6% dO₂. The method may further comprise maintaining the dO₂ level of the solution at at least about 8% dO₂ (e.g. at a level of at least about 10% dO₂ or at a level of at least about 12% dO₂) for at least about 10 minutes prior to commencing the filtering. The method may further comprise maintaining the dO₂ level of the solution at at least about 6% dO₂ for at least about 10 minutes prior to commencing the filtering. The method may further comprise maintaining the dO₂ level of the solution at at least about 8% dO₂ (e.g. at a level of at least about 10% dO₂ or at a level of at least about 12% dO₂) for at least about 15 minutes prior to commencing the filtering. The method may further comprise maintaining the dO₂ level of the solution at at least about 6% dO₂ for at least about 15 minutes prior to commencing the filtering.

The specified level may be a level of at least about 0.4 mg/L dO₂, e.g. at a level of at least about 0.6 mg/L dO₂ or at a level of at least about 0.8 mg/L dO₂. The filtering may be performed with the solution comprising a dissolved oxygen (dO₂) level of at least about 0.3 mg/L dO₂. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 10 minutes prior to commencing the filtering. The method may further comprise maintaining the dO₂ level of the solution at at least about 0.3 mg/L dO₂ for at least about 10 minutes prior to commencing the filtering. The method may further comprise maintaining the dO₂ level of the solution at at least about 0.4 mg/L dO₂ (e.g. at a level of at least about 0.6 mg/L dO₂ or at a level of at least about 0.8 mg/L dO₂) for at least about 15 minutes prior to commencing the filtering. The method may further comprise maintaining the dO₂ level of the solution at at least about 0.3 mg/L dO₂ for at least about 15 minutes prior to commencing the filtering.

In an embodiment, the dO₂ levels are maintained at at least the required level by oxygenating the solution and / or Optimizing the filtering. The required level may be about 6% dO₂, about 8% dO₂, about 10% dO₂ or about 12% dO₂. The required level may be about 8% dO₂. The required level may be about 0.3 mg/L dO₂, about 0.4 mg/L dO₂, about 0.6 mg/L dO₂, or about 0.8 mg/L dO₂. The required level may be about 0.4 mg/L dO₂.

The oxygenating may comprise sparging the solution with a gas comprising oxygen. The oxygenating may comprise agitating and/or mixing the solution to increase contact of the solution with gas comprising oxygen. The oxygenating may comprise sparging the solution with a gas comprising oxygen and agitating or mixing the solution to increase contact of the solution with gas comprising oxygen. The gas comprising oxygen may be atmosphere.

Optimizing the filtering may comprise increasing filtration rate, and/or filter size, and/or filter capacity, such that the dO₂ levels do not fall below a specified level during filtering. The specified level may be about 6% dO₂, about 8% dO₂, about 10% dO₂ or about 12% dO₂. The specified level may be about 8% dO₂. The specified level may be about 0.3 mg/L dO₂, about 0.4 mg/L dO₂, about 0.6 mg/L dO₂, or about 0.8 mg/L dO₂. The specified level may be about 0.4 mg/L dO₂.

The increasing may comprise increasing the filtration rate (and optionally the filter size, and/or optionally the filter capacity) by at least about 10%. The increasing may comprise increasing the filtration rate (and optionally the filter size, and/or optionally the filter capacity) by at least about 15%. The increasing may comprise increasing the filtration rate (and optionally the filter size, and/or optionally the filter capacity) by at least about 20%. The increasing may comprise increasing the filtration rate (and optionally the filter size, and/or optionally the filter capacity) by at least about 25%. The increasing may comprise increasing the filtration rate (and optionally the filter size, and/or optionally the filter capacity) by at least about 30%. The increasing may comprise increasing the filtration rate (and optionally the filter size, and/or optionally the filter capacity) by at least about 50%. The increasing may comprise increasing the filtration rate (and optionally the filter size, and/or optionally the filter capacity) by at least about 75%. The increasing may comprise increasing the filtration rate (and optionally the filter size, and/or optionally the filter capacity) by at least about 100%. The increasing may comprise increasing the filter size (and optionally the filter capacity) by at least about 10%. The increasing may comprise increasing the filter size (and optionally the filter capacity) by at least about 15%. The increasing may comprise increasing the filter size (and optionally the filter capacity) by at least about 20%. The increasing may comprise increasing the filter size (and optionally the filter capacity) by at least about 25%. The increasing may comprise increasing the filter size (and optionally the filter capacity) by at least about 30%. The increasing may comprise increasing the filter size (and optionally the filter capacity) by at least about 50%. The increasing may comprise increasing the filter size (and optionally the filter capacity) by at least about 75%. The increasing may comprise increasing the filter size (and optionally the filter capacity) by at least about 100%. The increasing may comprise increasing the filter capacity by at least about 10%. The increasing may comprise increasing the filter capacity by at least about 15%. The increasing may comprise increasing the filter capacity by at least about 20%. The increasing may comprise increasing the filter capacity by at least about 25%. The increasing may comprise increasing the filter capacity by at least about 30%. The increasing may comprise increasing the filter capacity by at least about 50%. The increasing may comprise increasing the filter capacity by at least about 75%. The increasing may comprise increasing the filter capacity by at least about 100%.

The method may comprise monitoring the level of oxygen prior to commencing the filtering, for example monitoring the level of oxygen for at least about 10 minutes (e.g. for at least about 15 minutes) prior to commencing the filtering. The method may comprise monitoring the level of dO₂ during filtering. The method may comprise monitoring the level of oxygen prior to commencing the filtering and monitoring the level of dO₂ during filtering. When the method comprises monitoring the level of oxygen, maintaining the level of oxygen at at least the specified level may comprise adding oxygen to the solution in response to the monitored level of oxygen approaching the specified level. When the method comprises monitoring the level of oxygen, maintaining the level of oxygen at at least the specified level may comprise increasing oxygenating the solution and/or optimizing the filtering in response to the monitored level of oxygen approaching the specified level. The level of dO₂ may be monitored using any suitable technique. The level of dO₂ may be monitored with an electrochemical oxygen sensor or an optical oxygen sensor. The level of dO₂ may be monitored with an electrochemical oxygen sensor. The level of dO₂ may be monitored with an optical oxygen sensor.

In embodiments, the cysteine is provided substantially as a cysteine derivative that does not comprise a sulfhydryl group. For example, the cysteine may comprise less than about 1 mM free cysteine, e.g. the cysteine may comprise less than 0.5 mM free cysteine (or less than 0.1 mM free cysteine). Cysteine derivatives that do not comprise sulfhydryl groups do not react with copper (II) to form insoluble copper (I).

The cysteine may be converted substantially into a cysteine derivative that does not comprise a sulfhydryl group prior to adding the copper (II) to the solution. The cysteine derivative may comprise a disulfide and/or a thiazolidine moiety.

The cysteine derivative may comprises a disulfide. For example, the cysteine derivative may comprise cystine, S-sulfocysteine, S-sulfocysteinylglycine, L-cysteine mixed disulfides, and/or S-sulfoglutathione. The cysteine derivative may comprise cystine.

The cysteine derivative may comprise a thiazolidine moiety. For example, the cysteine derivative may comprise 4-carboxy-2-methylthiazolidine-2-carboxylate, 2-methyl-1,3-thiazolidine-2,4-dicarboxylic acid, 2-(2-carboxyethyl)21,3-thiazolidine-2,4-dicarboxylic acid, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine). The cysteine derivative may comprise 4-carboxy-2-methylthiazolidine-2-carboxylate.

The cysteine derivative may comprise at least one of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g. FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid, or carbohydrate based thiazolidines (e.g. D-ribose-L-cysteine).

In embodiments, the copper may be in the form of a copper (II) chelate. The chelate may comprise at least one chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2).

In embodiments of any of the first, second or third aspects, the other components comprise at least one carbon source, and/or at least one nitrogen source, and/or at least one vitamin, and/or at least one buffering agent, and/or at least one inorganic salt, and/or at least one trace metal other than copper, and/or at least one polyamine, and/or at least one (essential) fatty acid, and/or at least one antioxidant, and/or at least one corticosteroid, and/or at least one chelator, and/or at least one lipase inhibitor. The other components may comprise at least one carbon source, and/or at least one nitrogen source, and/or at least one vitamin, and/or at least one buffering agent, and/or at least one inorganic salt, and/or at least one trace metal other than copper, and/or at least one polyamine, and/or at least one (essential) fatty acid. The other components may comprise at least one carbon source. The other components may comprise at least one nitrogen source. The other components may comprise at least one vitamin. The other components may comprise at least one buffering agent. The other components may comprise at least one inorganic salt. The other components may comprise at least one trace metal other than copper. The other components may comprise at least one polyamine. The other components may comprise at least one (essential) fatty acid. The other components may comprise at least one carbon source, at least one nitrogen source, at least one vitamin, at least one buffering agent, at least one inorganic salt, at least one trace metal other than copper, at least one polyamine, and at least one (essential) fatty acid.

The at least one carbon source may comprise a sugar, optionally a monosaccharide or disaccharide. The sugar may comprise at least one (e.g. two or more of) glucose, galactose, maltose, fructose, ribose and deoxyribose. The sugar may comprise glucose and/or deoxyribose.

The at least one nitrogen source (e.g. two or more nitrogen sources) may comprise at least one amino acid and/or ammonia/ammonium. The at least one nitrogen source may comprise at least one amino acid. The at least one amino acid may be selected from L-alanine, L-arginine, L-asparagine, L-aspartic acid, L-cystine, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine and L-valine.

The at least one vitamin (e.g. two or more vitamins) may be selected from ascorbic acid (e.g. ascorbic acid magnesium salt), biotin, choline (e.g. choline chloride), D-Ca2+-pantothenate, folic acid, inositol, menadione, niacinamide, nicotinic acid, paraaminobenzoic acid (PABA), pyridoxal, pyridoxine, riboflavin, thiamine, vitamin A acetate, vitamin B12, vitamin D2 and substances with vitamin-like activity (e.g.alpha lipoic acid or dihydrolipoic acid (DHLA)).

The at least one buffering agent may comprise at least one buffering agent (e.g. two or more buffering agents) that can buffer over a pH in the range of about 6 to about 8, such as a Good's buffer (e.g. as disclosed in N.E. Good, et al., "Hydrogen Ion Buffers for Biological Research", Biochemistry (1966),5(2), 467-477; N.E. Good and S. Izawa, "Hydrogen Ion Buffers, Methods in Enzymology, (1972), Vol. 24, 53-68; and W.J. Ferguson, et al., "Hydrogen Ion Buffers for Biological Research", Analytical Biochemistry, (1980), 104(2), 300-310). The at least one buffering agent may comprise at least one buffering agent (e.g. two or more buffering agents) selected from at least one buffering agent comprises carbonate, bicarbonate, phosphate, hydrogen phosphate, dihydrogen phosphate, lactate, 4-(2-hydroxyethyl)-1-piperazineethanesulfonic acid (HEPES), 3-(N-morpholino)propanesulfonic acid (MOPS), N,N-Bis(2-hydroxyethyl)-2-aminoethanesulfonic acid (BES), 2-[[1,3-dihydroxy-2-(hydroxymethyl)propan-2-yl]amino]ethanesulfonic acid (TES), 2-(N-morpholino)ethanesulfonic acid (MES), and 2-[Bis(2-hydroxyethyl)amino]-2-(hydroxymethyl)propane-1,3-diol (Bis-Tris).

The at least one inorganic salt (e.g. two or more inorganic salts) may comprise at least one soluble salt of calcium, potassium, magnesium, sodium, or iron. A soluble salt may be a salt having a solubility in water at 25°C of at least 1 g/L. The at least one inorganic salt (e.g. two or more inorganic salts) may comprises at least one of CaCl₂, KCl, MgCl₂, MgSO₄, NaCl, NaHCO₃, Na₂HPO₄, NaH₂PO₄ and ferric citrate chelate, or ferrous sulfate chelate.

The at least one trace metal (e.g. two or more trace metals) other than copper may comprise ions of at least one of barium, bromium, cobalt, iodine, manganese, chromium, nickel, selenium, vanadium, titanium, germanium, molybdenum, silicon, iron, fluorine, silver, rubidium, tin, zirconium, cadmium, zinc and aluminium.

The at least one polyamine (e.g. two or more polyamines) may comprise at least one of putrescine, cadaverine, spermine, and spermidine.

The at least one (essential) fatty acid (e.g. at least two fatty acids) may be selected from n-3, n-6, n-9 fatty acids and cholesterol. The at least one (essential) fatty acid (e.g. at least two fatty acids) may be selected from linoleic acid (ALA), linolenic acid (LA), doscosahexenoic acid (DHA), eicosapentaenoic acid (EPA) and arachidonic acid (AA). For example, the at least one essential fatty acid (e.g. at least two fatty acids) may be selected from ALA and linolenic acid LA.

The at least one antioxidant (e.g. two or more antioxidants) may comprise at least one of ascorbic acid, taurine, hypotaurine, a tocopherol, and a tocotrienol.

The at least one corticosteroid (e.g. two or more corticosteroids) may comprise at least one glucocorticoid (e.g. hydrocortisone) or mineralocorticoid (e.g. aldosterone).

The at least one metal chelator (e.g. two or more chelators) may comprise at least one of ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2), 1,2- bis(2-Aminophenoxy)ethane-N,N,N',N'-tetraacetic acid (BAPTA), 1,2-Bis(2-aminophenoxy)ethane-N,N,N',N'-tetraacetic acid tetrakis(acetoxymethyl ester) (BAPTA-AM), deferoxamine mesylate, .

In a fourth aspect, the disclosure provides a method of culturing a cell in a medium. The method comprises preventing loss of copper in the medium according to a method of the first aspect, or preparing the medium according to a method of the second or third aspect; contacting the cell with the medium; and culturing the cell in the medium.

The cell may be a eukaryotic, a prokaryotic or an archaea cell. The eukaryotic cell may be a mammalian cell. The cell may be selected from hybridoma cells, CHO cells, COS cells, VERO cells, HeLa cells, HEK 293 cells, PER-C6 cells, K562 cells, MOLT-4 cells, MI cells, NS-1 cells, COS-7 cells, MDBK cells, MDCK cells, MRC-5 cells, WI-38 cells, WEHI cells, SP2/0 cells, BHK cells (including BHK-21 cells) and derivatives thereof.

The culturing the cell in the medium may comprise multiplying the cell. The method may further comprise isolating and/or purifying the multiplied cell.

In a fifth aspect, the disclosure provides a method of making a biological product. The method comprises preventing loss of copper in the medium according to the first aspect or preparing a medium according to the second or third aspect; contacting a cell configured to express said biological product with the medium; and culturing the cell in the medium under conditions such that the cell expresses the biological product.

The biological product may be a polypeptide, protein, peptide, hormone, virus or virus like particle, nucleic acid or a fragment thereof; optionally an antibody, or a biologically functional fragment of an antibody.

The cell may be a eukaryotic, a prokaryotic or an archaea cell. The eukaryotic cell may be a mammalian cell. The cell may be selected from hybridoma cells, CHO cells, COS cells, VERO cells, HeLa cells, HEK 293 cells, PER-C6 cells, K562 cells, MOLT-4 cells, MI cells, NS-1 cells, COS-7 cells, MDBK cells, MDCK cells, MRC-5 cells, WI-38 cells, WEHI cells, SP2/0 cells, BHK cells (including BHK-21 cells) and derivatives thereof.

The method may further comprise isolating and/or purifying the product.

In a sixth aspect, the method provides a cell culture medium, cell culture feed, or cell culture additive obtainable or obtained by a method of the second or third aspect.

In a seventh aspect, the disclosure provides use of a cell culture medium, cell culture feed, or cell culture additive of the sixth aspect for culturing cells. The culturing may comprise producing a biological product from said cells. The culturing may comprise multiplying said cells. The culturing may comprise multiplying said cells and producing a biological product from said cells. The use may further comprise isolating the biological product.

### BRIEF DESCRIPTION OF THE DRAWINGS

Embodiments of the invention are further described hereinafter with reference to the accompanying drawings, in which:
Figure 1 illustrates a time course of copper removal by filtration in Genentech Essential Medium. Aliquots (n=3) were filtered at each time-point and the copper content was compared to unfiltered media.
Figure 2 provides an illustration of how several parameters varied over time in exemplary media after trace metals addition. a) provides the concentration profile for cysteine monomer, b) provides the profile for dissolved oxygen (dO₂) concentration compared to % saturation, c) provides the redox potential, and d) provides an overlay of the results for copper removal, cysteine monomer, and dissolved oxygen, allowing comparison of the relative levels of each of these parameters.
Figure 3 illustrates copper removal extended by a) increasing the starting cysteine concentration in an unmixed system b) increasing the starting cysteine concentration in a mixed system c) sparging with nitrogen after cysteine depletion d) sparging with nitrogen prior to cysteine depletion.
Figure 4 indicates the condensation reaction of cysteine and pyruvate to form a 2,4 thiazolidine.
Figure 5 provides cysteine monomer profiles measured by Ellman's reagent in simplified media, with 4 conditions compared: Simplified media without metal ions or pyruvate, with pyruvate, with metal ions, and with pyruvate and metal ions.
Figure 6 provides replicate data showing consistency of copper removal, cysteine monomer, dissolved oxygen, and redox profiles of cysteine containing cell culture media (compare with Figures 1 and 2).

### DETAILED DESCRIPTION

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

All publications, patent applications, patents, and other references mentioned herein are incorporated by reference in their entirety. In case of conflict, the present specification, including definitions, will control.

### DEFINITIONS

The following explanations of terms and methods are provided to better describe the present disclosure and to guide those of ordinary skill in the art in the practice of the present disclosure.

The term "soluble copper" as used herein includes reference to coper ions that are solvated, i.e. copper ions in solution that are surrounded or complexed by solvent molecules. The solution may be an aqueous solution, optionally comprising polar organic solvents (such as ethanol). The soluble copper may comprise or consist of copper (II) ions, e.g. copper (II) salt(s) having a solubility in water of greater than 1 g per 100 mL at 20 °C.

The term "insoluble" as used herein includes reference to solids (e.g. species comprising copper (I)) that have a solubility in water of less than 0.05 g per 100 mL at 20 °C.

The term "solution" as used herein, unless the context requires otherwise, includes reference to a liquid phase containing more than one substance. When the solution is a cell culture medium, cell culture feed, or cell culture additive, the solvent is typically water and the other substances in the solution are typically solutes. Solutes may be lost from the solution by precipitation.

The term "filtering" as used herein includes reference to the process of passing a solution through a porous material (filter). Solid particles present in the solution are retained on the filter, in particular when the particles are larger than the pores of the filter. Filtering of cell culture medium, cell culture feed, or cell culture additive may be performed using a filter with a pore size of not more than about 0.5 µm, such as not more than about 0.2 µm (e.g. not more than about 0.1 µm). The term "sterile filtering" as used herein refers to filtering performed with a filter having a pore size that is small enough to remove micororganisms, such as viruses, bacteria, fungi, mycoplasma, and transmissible spongiform encephalopathy agents, from the solution. Sterile filtering may use nanofilters with a pore size of not more than about 0.1 µm, such as a pore size of not more than 0.05 µm. Sterile filtering may provide a log reduction value (LRV) of relevant microorganisms of greater than about 4 LRV.

The term "filterable" with reference to a solution component, such as copper, means that the component is fully solvated and / or has a particle size that is sufficiently small for the particles to quantitatively pass through the pores of filter medium during filtering. A filterable component may comprise particles having a largest dimension of not more than 90% (e.g. not more than 70%) of the pore size of the filter. A filterable component may consist or comprise of fully solvated species and optionally particles having a largest dimension of not more than 90% (e.g. not more than 70%) of the pore size of the filter.

The term "dissolved oxygen" (dO₂) as used herein includes reference to the level of oxygen dissolved in a solution. The dO₂ may be provided in relative units of "% dO₂", which represents the percentage saturation of O₂ in water, based on an O₂ partial pressure of 212.2 mbar at the temperature of the solution (such as a temperature in the range of about 8 °C to about 40 °C). For example, the dO₂ may be at least about 8% dO₂. The dO₂ may be provided in absolute units of "mg/L". For example, the dO₂ may be at least about 1 mg/L at a temperature of 8 °C, and/or the dO₂ may be at least about 0.3 mg/L at a temperature of 40 °C.

The term "atmosphere" as used herein includes reference to air having a composition that is similar to that of the Earth's atmosphere at sea level, comprising nitrogen, oxygen, water vapour, argon, carbon dioxide and trace gasses. The level of oxygen in dry atmosphere is typically about 20 to about 21% by volume.

The term "chelate" as used herein refers to complexes comprising a metal ion and at least one multidentate (e.g. bidentate, tridentate, tetradentate, petadentate, hexadentate, etc.) ligand. An exemplary metal ion in copper (II). Exemplary multidentate ligands include ethylenediaminetetraacetate (EDTA), diethylenetriamine pentaacetate (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2). While the exemplary multidentate ligands are indicated in a particular acid or base form, it will be appreciated that the ligands may be provided in any suitable conjugate acid or conjugate base forms.

The term "cysteine" as used herein includes reference to the amino acid L-cysteine, tautomer, geometric isomer, salt or solvate thereof: 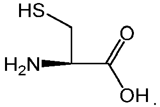 A "cysteine derivative" as used herein includes reference to a derivative of cystine where the hydrogen of the disulfide has been replaced with a bond to another moiety, so the cysteine derivative does not comprise a sulfhydryl group. For example, the cysteine derivative may comprise a disulfide or a thiazolidine moiety.

The term "cell culture medium" as used herein refers to a nutritive solution for cultivating cells. A "cell culture feed" and a "cell culture additive" represent nutritive supplements that may be added to a cell culture medium to improve medium performance. For example, a cell culture feed and/or a cell culture additive may be added to a cell culture medium during batch culture of cells. A cell culture medium may be chemically defined or may comprise undefined components.

The term "contacting" as used herein includes reference to the placing of cells to be cultivated in vitro into a culture vessel with the medium in which the cells are to be cultivated. The term "contacting" encompasses mixing cells with medium, pipetting medium onto cells in a culture vessel, and submerging cells in culture medium.

The term "combining" as used herein refers to the mixing or admixing of ingredients in a cell culture medium formulation.

A "chemically defined" medium as used herein is a medium in which every ingredient is known. A chemically defined medium is distinguished from serum, embryonic extracts, and hydrolysates, each of which contain unknown components. A cell culture medium of the present disclosure may be a chemically defined medium. A cell culture feed of the present disclosure may be chemically defined. A cell culture additive of the present disclosure may be chemically defined.

An "undefined medium" or "medium comprising undefined component(s)" as used herein includes reference to a medium that comprises one or more ingredients that are not known. Undefined components may be provided by, for example, serum, peptones, hydrolysates (such as yeast, plant or serum hydrolysate), and embryonic extracts.

The term "ingredient" as used herein refers to any compound, whether of chemical or biological origin, that can be used in cell culture media, feed or additives, to maintain or promote the growth or proliferation of cells, or the expressions of product by the cells. The terms "component", "nutrient", and "ingredient" may be used interchangeably and are all meant to refer to such compounds. Typical ingredients that are used in cell culture media include carbon sources (such as simple carbohydrates, e.g. sugars), nitrogen sources (such as amino acids), vitamins, buffering agents, inorganic salts, trace metals, polyamines, lipdis, fatty acids. Exemplary ingredients include amino acids, salts, metals, sugars, lipids, nucleic acids, hormones, vitamins, fatty acids, proteins and the like. Other ingredients that promote or maintain cultivation of cells ex vivo can be selected by those of skill in the art, in accordance with the particular need.

A cell culture medium is composed of a number of ingredients and these ingredients vary from one culture medium to another. A "1X formulation" as used herein is meant to refer to any aqueous solution that contains some or all ingredients found in a cell culture medium at working concentrations. The "1X formulation" can refer to, for example, the cell culture medium or to any subgroup of ingredients for that medium. The concentration of an ingredient in a 1X solution is about the same as the concentration of that ingredient found in a cell culture formulation used for maintaining or cultivating cells in vitro. A cell culture medium used for the in vitro cultivation of cells is a 1X formulation by definition. When a number of ingredients are present, each ingredient in a 1X formulation has a concentration about equal to the concentration of those ingredients in a cell culture medium. Thus, when referring to a "1X formulation," it is intended that each ingredient in solution has the same or about the same concentration as that found in the cell culture medium being described. The concentrations of ingredients in a 1X formulation of cell culture medium are well known to those of ordinary skill in the art, with the relevant components and concentrations of the components dependent on the cell type and application. See, for example, Methods For Preparation of Media, Supplements and Substrate For Serum-Free Animal Cell Culture, New York: Allen R. Liss (1984); H.J. Morton, "A survey of commercially available tissue culture media", In Vitro (1970), 6(2), 89-108; and J. Van der Valk, et al., (2010), "Optimization of chemically defined cell culture media-replacing fetal bovine serum in mammalian in vitro methods," Toxicology in vitro, 24(4), 1053-1063; all of which are incorporated by reference herein in their entirety. The osmolarity and/or pH, however, may differ in a 1X formulation compared to the culture medium, particularly when fewer ingredients are contained in the 1X formulation.

An "nnX formulation" as used herein, where "nn" is an integer greater than 1 refers to a solution where each ingredient in that solution is provided about nn time more concentrated than the corresponding ingredient in a 1X formulation. Cell culture feeds and cell culture additives may be provided as 1X to 1.5 x 10⁹X formulations, e.g. as 1X to 10,000X formulations. For example, a cell culture feed may be provided as a 1X to 1,000X formulation. As the skilled person will appreciate, the upper "nnX" limit for a given formulation will be dependent on the solubility of each component in the formulation and the required concentration of each component in the corresponding 1X formulation. Thus a higher "nn" is typically possible for feeds and additives that comprise only components with a relatively high solubility that are also required at a relatively low concentration in the corresponding 1X formulation.

The term "cell" as used herein includes reference to a eukaryotic cell, a prokaryotic cells and an archaea cell. Unless the context requires otherwise, reference to a cell may include reference to the plural (cells). A eukaryotic cell may be a mammalian cell, such as a hybridoma, CHO cell, COS cell, VERO cell, HeLa cell, HEK 293 cell, PER-C6 cell, K562 cell, MOLT-4 cell, MI cell, NS-1 cell, COS-7 cell, MDBK cell, MDCK cell, MRC-5 cell, WI-38 cell, WEHI cell, SP2/0 cell, BHK cell (including BHK-21 cell) and derivatives thereof. A prokaryotic cell may be an *Eschericia coli, pseudomonas sp., bacillus sp., Streptomyces sp., lactobacillus sp., lactoccocus sp.,* and derivatives thereof. An archaea cell may be a Haloarchaea and derivatves thereof.

### CELL CULTURE MEDIA

Cell culture media contain many components. Cell culture media provide the nutrients necessary to maintain and grow cells in a controlled, artificial and *in vitro* environment. Characteristics and compositions of the cell culture media vary depending on the particular cellular requirements. Important parameters include osmolarity, pH, and nutrient formulations.

Culture media contain a mixture of amino acids, glucose, salts, vitamins, and other nutrients, and are available either as a powder or as a liquid form from commercial suppliers. The requirements for these components vary among cell lines. Regulation of pH is critical for optimum culture conditions and is generally achieved using a suitable buffering system. While CDM are preferred for therapeutic and related applications, as CDM provide reproducible contamination-free media when prepared and used under aseptic conditions, for some cell types it may be necessary to use media comprising serum, proteins or other biological extracts (such as yeast extracts or enzymatic digests of plant or animal matter).

Some extremely simple defined media, which consist essentially of vitamins, amino acids, organic and inorganic salts and buffers have been used for cell culture. Such media (often called "basal media"), however, are usually seriously deficient in the nutritional content required by most animal cells. These media therefore often need to be supplemented, for example with feeds or other additives, to form complete media. In addition, batch culture systems often include periodic supplementation of the media with concentrated feeds or additives, to maintain the viability of cultured cells and/or production of biological products, such as polypeptides (e.g. antibodies, or biologically functional fragments of antibodies), proteins, peptides, hormones, viruses or virus like particles, nucleic acids or fragments thereof.

Ingredients that may be present in basal media include amino acids (nitrogen source), vitamins, inorganic salts, sugars (carbon source), buffering salts and lipids. Basal media for use with some mammalian cell culture systems may contain ethanolamine, D-glucose, N-[2-hydroxyethyl]-piperazine-N'-[2-ethanesulfonic acid] (HEPES), linoleic acid, lipoic acid, phenol red, PLURONIC F68, putrescine, sodium pyruvate.

Amino acid ingredients which may be included in the media include L-alanine, L-arginine, L-asparagine, L-aspartic acid, L-cysteine, L-glutamic acid, L glutamine, glycine, L-histidine, L-isoleucine, L-leucine, L-lysine, L-rnethionine, L phenylalanine, L-proline, L-serine, L-threonine, L-tryptophan, L-tyrosine, L-valine, and derivatives thereof. These amino acids may be obtained commercially, for example from Sigma (Saint Louis, Missouri).

Vitamin ingredients which may be included in the media include biotin, choline chloride, D-Ca²⁺-pantothenate, folic acid, i-inositol, niacinamide, pyridoxine, riboflavin, thiamine and vitamin B12. These vitamins may be obtained commercially, for example from Sigma (Saint Louis, Missouri).

Inorganic salt ingredients which may be used in the media include one or more calcium salts (*e.g.*, CaCl₂), Fe(NO₃)₃, KCl, one or more magnesium salts (*e.g.,* MgCl2 and/or MgSO₄), one or more manganese salts (*e.g.,* MnCl₂), NaCl, NaHCO₃, N₂HPO₄, and ions of the trace elements selenium, vanadium, zinc and copper. These trace elements may be provided in a variety of forms, preferably in the form of salts such as Na₂SeO₃, NH₄VO₃, ZnSO₄ and CuSO₄. These inorganic salts and trace elements may be obtained commercially, for example from Sigma (Saint Louis, Missouri).

Exemplary media that are useful in the culture of mammalian include commercially available media such as Ham's F10 (Sigma), Minimal Essential Medium ((MEM), Sigma), RPMI-1640 (Sigma), and Dulbecco's Modified Eagle's Medium ((DMEM, Sigma) are suitable for culturing the host cells. In addition, any of the media described in Ham and Wallace (1979), Meth. in Enz. 58:44; Barnes and Sato (1980), Anal. Biochem. 102:255; U.S. Pat. No. 4,767,704; 4,657,866; 4,927,762; or 4,560,655; WO 90/03430; WO 87/00195; U.S. Pat. No. Re. 30,985; or U.S. Pat. No. 5,122,469, the disclosures of all of which are incorporated herein by reference, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan. Exemplary culture conditions are provided in M. Takagi and K. Ueda, "Comparison of the optimal culture conditions for cell growth and tissue plasminogen activator production by human embryo lung cells on microcarriers", Biotechnology, (1994), 41, 565-570; H.J. Morton, "A survey of commercially available tissue culture media", In Vitro (1970), 6(2), 89-108; J. Van der Valk, et al., (2010), "Optimization of chemically defined cell culture media-replacing fetal bovine serum in mammalian in vitro methods," Toxicology in vitro, 24(4), 1053-1063; R.J. Graham et al., "Consequences of trace metal variability and supplementation on Chinese hamster ovary (CHO) cell culture performance: A review of key mechanisms and considerations", Biotechnol. Bioeng. (2019), 116(12), 3446-3456; S. Janoschek et al., A protocol to transfer a fed-batch platform process into semi-perfusion mode: The benefit of automated small-scale bioreactors compared to shake flasks as scale-down model", Biotechnol. Prog., (2019), 35(2), e2757; and M. Kuiper et al., "Repurposing fed-batch media and feeds for highly productive CHO perfusion processes", Biotechnology Progress, 15 April 2019, https://doi.org/10.1002/btpr.2821; all of which are incorporated by reference herein in their entirety

The cell culture media, feeds and additives of the disclosure and invention comprise copper (II) and cysteine. The amounts of copper (II) and cysteine (including cysteine derivatives) present in exemplary media, feeds and additives is indicated in table 1.

**Table 1: Exemplary concentrations of copper (II) and cysteine**

| **Media Type** | **Component** | **Exemplary concentrations (mM)** |
|---|---|---|
| Basal medium | Cysteine | 0.25 - 12 |
| Basal medium | Copper (II) | 0.0000001 - 0.01 |
| Feed | Cysteine | 1 - 50 |
| Feed | Copper (II) | 0.1 - 25 |
| Additive | Cysteine | 10 - 2000 |
| Additive | Copper (II) | 1 - 1000 |

The cysteine may be provided as L-cysteine may be provided in multiple hydration and/or salt forms. These cysteine containing forms may be solubilized in water, organic solvent, basic solvent, or acidic solvent to enhance solubility if the solvent when at process working concentrations in contact with the culture and/or product does not impact cell culture performance or product quality. For certain cell types, at least a portion of the cysteine (e.g. substantially all the cysteine) may be provided as a cysteine derivative. For example, as disclosed herein substantially all of the cysteine may be provided as a cysteine derivative that does not comprise a sulfhydryl group. The copper (II) may be provided in multiple hydration and/or salt forms (typically sulfate salts with some water content). These copper containing forms may be solubilized in water, organic solvent, basic solvent, or acidic solvent to enhance solubility if the solvent when at process working concentrations in contact with the culture and/or product does not impact cell culture performance or product quality.

Other components of the cell culture media, feeds and additives comprise at least one carbon source, and/or at least one nitrogen source, and/or at least one vitamin, and/or at least one buffering agent, and/or at least one inorganic salt, and/or at least one trace metal other than copper, and/or at least one polyamine, and/or at least one (essential) fatty acid. The other components may comprise at least one carbon source. The other components may comprise at least one nitrogen source. The other components may comprise at least one vitamin. The other components may comprise at least one buffering agent. The other components may comprise at least one inorganic salt. The other components may comprise at least one trace metal other than copper. The other components may comprise at least one polyamine. The other components may comprise at least one (essential) fatty acid. The other components may comprise at least one carbon source, at least one nitrogen source, at least one vitamin, at least one buffering agent, at least one inorganic salt, at least one trace metal other than copper, at least one polyamine, and at least one (essential) fatty acid.

The at least one carbon source may comprise a sugar, optionally a monosaccharide or disaccharide. The sugar may comprise at least one (e.g. two or more of) glucose, galactose, maltose, fructose, ribose and deoxyribose. The sugar may comprise glucose and/or deoxyribose.

The at least one nitrogen source (e.g. two or more nitrogen sources) may comprise at least one amino acid and/or ammonia/ammonium. The at least one nitrogen source may comprise at least one amino acid. The at least one amino acid may be selected from L-alanine, L-arginine, L-asparagine, L-aspartic acid, L-cystine, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine and L-valine, or derivatives thereof. In embodiments, the at least one amino acid includes but is not limited to L-alanine, L-arginine, L-asparagine, L-aspartic acid, L-cystine, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine, or derivatives thereof.

The at least one vitamin (e.g. two or more vitamins) may be selected from ascorbic acid (e.g. ascorbic acid magnesium salt), biotin, choline (e.g. choline chloride), D-Ca2+-pantothenate, folic acid, inositol, menadione, niacinamide, nicotinic acid, paraaminobenzoic acid (PABA), pyridoxal, pyridoxine, riboflavin, thiamine, vitamin A acetate, vitamin B12 and vitamin D2.

The at least one buffering agent may comprise at least one buffering agent (e.g. two or more buffering agents) that can buffer over a pH in the range of about 6 to about 8, e.g. a Good's buffer (e.g. as disclosed in N.E. Good, et al., "Hydrogen Ion Buffers for Biological Research", Biochemistry (1966),5(2), 467-477; N.E. Good and S. Izawa, "Hydrogen Ion Buffers, Methods in Enzymology, (1972), Vol. 24, 53-68; and W.J. Ferguson, et al., "Hydrogen Ion Buffers for Biological Research", Analytical Biochemistry, (1980), 104(2), 300-310). The at least one buffering agent may comprise at least one buffering agent (e.g. two or more buffering agents) selected from at least one buffering agent comprises carbonate, bicarbonate, phosphate, hydrogen phosphate, dihydrogen phosphate, lactate, 4-(2-hydroxyethyl)-1-piperazineethanesulfonic acid (HEPES), 3-(N-morpholino)propanesulfonic acid (MOPS), N,N-Bis(2-hydroxyethyl)-2-aminoethanesulfonic acid (BES), 2-[[1,3-dihydroxy-2-(hydroxymethyl)propan-2-yl]amino]ethanesulfonic acid (TES), 2-(N-morpholino)ethanesulfonic acid (MES), and 2-[Bis(2-hydroxyethyl)amino]-2-(hydroxymethyl)propane-1,3-diol (Bis-Tris).

The at least one inorganic salt (e.g. two or more inorganic salts) may comprise at least one soluble salt of calcium, potassium, magnesium, sodium, or iron. A soluble slat may be a salt having a solubility in water at 25°C of at least 1 g/L. The at least one inorganic salt (e.g. two or more inorganic salts) may comprises at least one of CaCl₂, KCl, MgCl₂, MgSO₄, NaCl, NaHCO₃, Na₂HPO₄, NaH₂PO₄ and ferric citrate chelate, or ferrous sulfate chelate. The at least one trace metal (e.g. two or more trace metals) other than copper may comprise ions of at least one of barium, bromium, cobalt, iodine, manganese, chromium, nickel, selenium, vanadium, titanium, germanium, molybdenum, silicon, iron, fluorine, silver, rubidium, tin, zirconium, cadmium, zinc and aluminium.

The at least one polyamine (e.g. two or more polyamines) may comprise at least one of putrescine, cadaverine, spermine, and spermidine.

The at least one (essential) fatty acid (e.g. at least two fatty acids) may include, but not be limited to, n-3, n-6, n-9 fatty acids and cholesterol. The at least one (essential) fatty acid (e.g. at least two fatty acids) may include but not be limited to linoleic acid (ALA), linolenic acid (LA), doscosahexenoic acid (DHA), eicosapentaenoic acid (EPA) and arachidonic acid (AA), and the like. The at least one essential fatty acid (e.g. at least two fatty acids) may be selected from linoleic acid and linolenic acid.

An aspect provides cell culture media, cell culture feed, or cell culture additive comprising copper (II) and cysteine, obtained or obtainable according to a method of the invention.

### METHODS OF PREVENTING COPPER LOSS AND/OR PREPARING MEDIA

Cell culture media, feed or additives, may lose copper during preparation. In particular, when cell culture media, feed or additives are filtered (e.g. by sterile filtration) insoluble copper species (e.g. copper (I) species) may be retained on the filter. This is problematic, as filtration, in particular sterile filtration, represents a widely used method for providing aseptic media, feeds and additives, as these compositions typically contain components that are degraded by terminal sterilization.

We have determined that cysteine, which is often present in cell culture medium, has significant involvement in causing copper filtration removals. For example, when we tested modified media that did not contain cysteine, no copper loss is observed. In media that uses cystine (the oxidized dimer form) rather than cysteine, copper removal during filtration was also not observed. We also observed that the time course transience of the copper loss was influenced by iron and pyruvate. The copper loss was pH dependent, occurring most severely around neutral pH, but was not restricted to a specific buffer species. It should be noted that, as cysteine is typically present in significant excess compared to copper, the relatively modest loss of cysteine from the media/feed/additive typically has little effect on the overall levels of cysteine in the solution, whereas the loss of copper typically has a more significant effect.

Cysteine oxidizes to its dimer form cystine in the presence of metal ion catalysts and oxygen (Cavallini D, et al., The copper catalyzed oxidation of cysteine to cystine. Archives of biochemistry and biophysics. 1969;130(1):354-361, which is incorporated by reference herein in its entirety). The oxidation of cysteine occurs according to the following:

*2RSH + O*₂ → *RSSR + H*₂*O*₂ (1)

*2RSH + H*₂*O*₂ → *RSSR +* 2*H*₂*O* (2)

Providing the net reaction:

*4 RSH + O*₂ → *2 RSSR +* 2*H*₂*O* (3)

*"RSH"* is cysteine and *"RSSR"* is cystine.

In the first reaction cysteine reacts with oxygen to generate cystine and hydrogen peroxide and in the second the hydrogen peroxide reacts with a second equivalent of cysteine to yield an additional cystine and two water equivalents. Both copper and iron catalyze cysteine oxidation, however copper is reported to be the more active catalyst (Munday R, et al., Inhibition of copper-catalyzed cysteine oxidation by nanomolar concentrations of iron salts. Free Radical Biology and Medicine. 2004;36(6):757-764; Ehrenberg L, et al., Kinetics of the copper-and iron-catalysed oxidation of cysteine by dioxygen. Acta Chemica Scandinavica. 1989;43, which are each incorporated by reference herein in their entirety). The first step in the catalysed reaction is believed to be a reductive chelation of the metal ion by cysteine, yielding a cysteine-Cu(I) or cysteine-Fe(ll) complex (Pecci L, et al., Novel findings on the copper catalysed oxidation of cysteine. Amino Acids. 1997;13(3-4):355-367, which is incorporated by reference herein in its entirety). Thus when there is cysteine monomer available to be oxidised, the metals ions will be reduced, in the case of copper to the copper (I) state.

Thus cysteine oxidation results in the formation of insoluble copper (I) species. That this occurs in cell culture media was confirmed with an assay for copper (I). In addition, we also determined that, in the presence of sufficient amounts of dO₂, the relatively insoluble copper (I) is readily oxidised back into the copper (II), which represents soluble copper.

The known chemistry is illustrated by the following reactions:

2*Cu⁺* + *O*₂ *+* 2*H⁺* → 2*Cu*²⁺ *+ H*₂*O*₂ (4)

2*Cu⁺ + H*₂*O*₂ *+* 2*H⁺* → 2*Cu*²*⁺ +* 2*H*₂*O* (5)

Providing the net reaction:

4*Cu⁺ + O*₂ *+* 4*H⁺* → 4*Cu*²*⁺ +* 2*H*₂*O* (6)

Without wishing to be bound by any theory, it is believed that a similar reaction would occur with the Cu(I)-cysteine complex to what occurs with free Cu(I) to decompose the catalyst and release Cu(ll). This mechanism provides an explanation for why dO₂ must be low for copper removal during filtration to occur and why oxygen is required for copper to become filterable again.

It follows that one way to avoid insoluble copper (I) is to ensure that there is sufficient dO₂ in the solution to prevent precipitation of copper (I) species. This may be achieved in a number of ways. For example, prior to and/or during filtration the level of oxygen may be kept at a sufficiently high specified level to ensure quantitative oxidation of any copper (I) to copper (II). The specified level may be at least about 6% dO₂, at least about 8% dO₂, at least about 10% dO₂ or at least about 12% dO₂. The specified level may be about 0.3 mg/L dO₂, about 0.4 mg/L dO₂, about 0.6 mg/L dO₂, or about 0.8 mg/L dO₂. Additionally (or alternatively) filtering may be optimized so that filtration is completed while there is still sufficient dO₂ in the solution to prevent precipitation of copper (I) species. For example, filtering may be optimised by increasing filtration rate, and/or filter size, and/or filter capacity, such that the dO₂ levels do not fall below a specified level during filtering.

Filtering may be optimised by manipulation of filtration unit operation variables to meet a fixed filtration time need for a given volume to be processed (e.g. manipulation of filter area, flow rates, etc. based on the method of filtration (e.g. dead end membrane) and by characterizing the system empirically or with empirical and theoretical approach, using techniques that are known to the skilled person. See, for example, Ozturk S and Hu W, Cell Culture Technology for Pharmaceutical and Cell-based Therapies, 2005; Rajniak P et al., Sterilizing filtration - Principles and practice for successful scale-up to manufacturing, 2008; the entire disclosures of which are expressly incorporated by reference herein.

For example, optimal filter size may be determined using empirical filter sizing studies, that can be performed based on various endpoints as a function of the relevant at-scale filtration unit operation (Pmax (pressure) or Tmax (turbidity) for constant flow, Vmax (volume) for constant pressure. This will allow one to define the filter area needed to maintain a flux that will process the batch volumes in a given time. There are practical limits, of course, at a giving production site/line based on space, cost, etc. Typical, normalized (unitless) fluxes used for scale up/scale down used for scaling in our industry can range from 10 to 8000 and cover processed media volumes from 50 L to 25000 L. These methods may also be extrapolated o cover larger or smaller volumes, when required.

The filtering may be performed using a filter with a pore size of not more than about 0.5 µm, such as not more than about 0.2 µm (e.g. not more than about 0.1 µm). The filtering may comprise sterile filtering, e.g. filtering performed with nanofilters with a pore size of not more than about 0.1 µm, such as a pore size of not more than 0.05 µm. Sterile filtering may provide a log reduction value (LRV) of relevant microorganisms (e.g. viruses) of greater than about 4 LRV. Nanofiltration of cell culture solutions in upstream operations, such as preparation of cell culture media, feeds and additives, represents an important approach for reducing microorganism contamination. See, for example Shirota, M. and Kiss, R., "Risk Mitigation in Preventing Adventitious Agent Contamination of Mammalian Cell Cultures", Adv. Biochem. Eng. Biotechnol., 2017, https://doi.org/10.1007/10_2017_38; and Miesegaes, G., et al., Virus retentive filters. In: Flickinger M (ed) Encyclopedia of industrial biotechnology: bioprocess, bioseparation, and cell technology, Wiley, 2010, pp 1-11, which are each incorporated by reference herein in their entirety. Filters include but are not limited to membrane filters (see, e.g. https://www.mrwa.com/WaterWorksMnI/Chapter%2019%20Membrane%20Filtration.pdf, incorporated herein by reference in its entirety), hollow fiber filters, column filters , spiral membrane filters , tube filters, capillary filters, capsule filters, cassette filters, disc filters, frit filters, and plug filters.

Any method that ensures that there is sufficient dO₂ in the solution to prevent precipitation of copper (I) species may comprise monitoring the level of oxygen prior to commencing the filtering, for example monitoring the level of oxygen for at least about 10 minutes (e.g. for at least about 15 minutes) prior to commencing the filtering. The method may comprise monitoring the level of dO₂ during filtering. The method may comprise monitoring the level of oxygen prior to commencing the filtering and monitoring the level of dO₂ during filtering. When the method comprises monitoring the level of oxygen, maintaining the level of oxygen at at least the specified level may comprise adding oxygen to the solution in response to the monitored level of oxygen approaching the specified level. When the method comprises monitoring the level of oxygen, maintaining the level of oxygen at at least the specified level may comprise increasing oxygenating the solution and/or optimizing the filtering in response to the monitored level of oxygen approaching the specified level. The level of dO₂ may be monitored using any suitable technique. The level of dO₂ may be monitored with an electrochemical oxygen sensor or an optical oxygen sensor. The level of dO₂ may be monitored with an electrochemical oxygen sensor. The level of dO₂ may be monitored with an optical oxygen sensor. Suitable oxygen sensors and methods for detecting dissolved oxygen are known to the skilled person. See, for example, A Guide to Oxygen Measurement: Theory and Practice of Oxygen Applications, Mettler-Toledo GmbH, 2016; Wei, Y., et al., "Review of dissolved oxygen detection technology: From laboratory analysis to online intelligent detection", Sensors, 2019, 19, 3995, which are each incorporated by reference herein in their entirety.

In addition to cysteine oxidation to cystine, cysteine in media may also be consumed by the condensation of cysteine and pyruvate to yield a 2,4-thiazolidine through a Schiff base intermediate (Sullivan MX and Hess WC, The Effect of Pyruvic Acid on the Estimation of Cystine and Cysteine, The Journal of Biological Chemistry, 1937, 122; Nishiuch Y, et al., Cytotoxicity of cysteine in culture media, In Vitro, 1976, 12(9); Rohac R, et al., Carbon-sulfur bond-forming reaction catalysed by the radical SAM enzyme HydE, Nature Chemistry, 2016, 8(5), 491-500; which are each incorporated by reference herein in their entirety). This reaction has been previously shown to occur in cysteine and pyruvate containing cell culture media with the 2,4 thiazolidine product confirmed by mass spectrometry (Kuschelewski J, et al., Antioxidant effect of thiazolidine molecules in cell culture media improves stability and performance, Biotechnology progress, 2017; which is incorporated by reference herein in its entirety). This provides another approach that can be used to prevent loss of copper. If the cysteine is reacted in a competing reaction, e.g. by condensation with pyruvate, it is not available to reduce copper (II) to insoluble copper (I) forms. Having said that, if the cysteine is in the presence of both copper (II) and pyruvate, both reactions may occur, so it is possible that some of the soluble copper (II) will still be lost from the solution.

If the cell culture media, feed or additive is intended for use with cells that are able to efficiently metabolise cysteine derivatives, it may be possible to avoid loss of soluble copper by providing cysteine substantially as a cysteine derivative that does not comprise a sulfhydryl group, prior to adding the copper (II) to the solution. For example, the cysteine may comprise less than about 1 mM free cysteine, e.g. the cysteine may comprise less than 0.5 mM free cysteine (or less than 0.1 mM free cysteine). Cysteine derivatives that do not comprise sulfhydryl groups do not react with copper (II) to form insoluble copper (I). This approach is, however, less suitable than other methods disclosed herein that prevent loss of soluble copper, where the media, feed or additive is intended for use with cells that do not efficiently metabolize such cysteine derivatives.

The cysteine derivative may comprise a disulfide, a thiazolidine moiety, or a protected sulfur group. The cysteine derivative may comprise a disulfide and/or a thiazolidine moiety. The cysteine derivative may comprises a disulfide. For example, the cysteine derivative may comprise cystine, S-sulfocysteine, S-sulfocysteinylglycine, L-cysteine mixed disulfides, and/or S-sulfoglutathione. The cysteine derivative may comprise cystine. The cysteine derivative may comprise a thiazolidine moiety. For example, the cysteine derivative may comprise 4-carboxy-2-methylthiazolidine-2-carboxylate, 2-methyl-1,3-thiazolidine-2,4-dicarboxylic acid, 2-(2-carboxyethyl)21,3-thiazolidine-2,4-dicarboxylic acid, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine). The cysteine derivative may comprise 4-carboxy-2-methylthiazolidine-2-carboxylate. The cysteine derivative may comprise at least one of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g. FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid, or carbohydrate based thiazolidines (e.g. D-ribose-L-cysteine).

The copper may be provided in the form of a copper (II) chelate. The chelate may comprise at least one chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2). Without wishing to be bound by any theory, it is considered that having the copper in the form of a copper (II) chelate, prevents appreciable reduction of the copper to copper (I), thereby avoiding loss of soluble copper from the solution.

A further approach to preventing loss of copper in cell culture medium, feed, or additive is to separately filter the copper (II) and cysteine solutions and then combine the two filtered solutions to form the final solution. This could be done providing the bulk of the reagents and volume in the cysteine solution, filtering that solution and then adding a bolus of filtered copper solution. This may be advantageous, as copper is a trace element typically provided at a relatively low concentration in the final solution. Another approach would be to provide copper and the bulk of the components in one filtered solution, then add the filtered cysteine solution to form the final solution. As the skilled person will appreciate, various permutations and combinations of separately filtering and then combining filtered solutions the final filtered media, feed or additive may be used in such methodologies, provided the copper (II) and cysteine solutions are filtered separately. In addition, where a final aseptic solution is desired, the filtered solutions should be handled in an aseptic manner.

This prevents any reaction between the copper (II) and cysteine prior to or during filtration that forms insoluble copper (I) species. While it is possible that the copper (I) species might form after filtration, this will remain in the medium, feed, or additive. Furthermore, as cell culture involves adding oxygen to the solution during culturing, any insoluble copper (I) would be oxidised to soluble copper (II) during culturing. Thus all of the copper in the medium, feed, or additive would be available for use by the cultured cells.

An aspect of the disclosure provides a method of preventing loss of copper in a solution. The solution is a cell culture medium, cell culture feed or cell culture additive comprising copper(II) and cysteine. The method comprises having substantially the same amount of soluble copper before and after filtering the solution.

Another aspect of the disclosure provides a method of preparing a cell culture medium, cell culture feed or cell culture additive comprising copper (II), cysteine, and other components, without loss of soluble copper. The method comprises providing an aqueous solution comprising cysteine and other components; providing an aqueous solution comprising copper (II); separately filtering the aqueous solution comprising cysteine and other components, and the aqueous solution comprising copper (II); and combining the filtered solutions to provide the cell culture medium, cell culture feed or cell culture additive.

A further aspect of the disclosure provides a method of preparing a cell culture medium, cell culture feed or cell culture additive comprising copper (II), cysteine, and other components, without loss of soluble copper. The method comprises filtering a solution comprising the copper (II), cysteine, and other components to provide the cell culture medium, cell culture feed or cell culture additive.

### METHODS OF CULTURING AND/OR MAKING BIOLOGICAL PRODUCTS

Also provided are methods of culturing cells and / making biological products from cultured cells.

A protocol for the production, recovery and purification of recombinant antibodies in mammalian, such as CHO, cells may include the following steps:
Cells may be cultured in a stirred tank bioreactor system and a fed batch culture, procedure is employed. In a preferred fed batch culture the mammalian host cells and culture medium are supplied to a culturing vessel initially and additional culture nutrients are fed, continuously or in discrete increments, to the culture during culturing, with or without periodic cell and/or product harvest before termination of culture. The fed batch culture can include, for example, a semi-continuous fed batch culture, wherein periodically whole culture (including cells and medium) is removed and replaced by fresh medium. Fed batch culture is distinguished from simple batch culture in which all components for cell culturing (including the cells and all culture nutrients) are supplied to the culturing vessel at the start of the culturing process. Fed batch culture can be further distinguished from perfusion culturing insofar as the supernate is not removed from the culturing vessel during the process (in perfusion culturing, the cells are restrained in the culture by, e.g., filtration, encapsulation, anchoring to microcarriers etc. and the culture medium is continuously or intermittently introduced and removed from the culturing vessel).

Further, the cells of the culture may be propagated according to any scheme or routine that may be suitable for the particular host cell and the particular production plan contemplated. Therefore, a single step or multiple step culture procedure may be employed. In a single step culture the host cells are inoculated into a culture environment and the processes are employed during a single production phase of the cell culture. Alternatively, a multi-stage culture can be used. In the multi-stage culture cells may be cultivated in a number of steps or phases. For instance, cells may be grown in a first step or growth phase culture wherein cells, possibly removed from storage, are inoculated into a medium suitable for promoting growth and high viability. The cells may be maintained in the growth phase for a suitable period of time by the addition of fresh medium to the host cell culture.

fed batch or continuous cell culture conditions may be devised to enhance growth of the mammalian cells in the growth phase of the cell culture. In the growth phase cells are grown under conditions and for a period of time that is maximized for growth. Culture conditions, such as temperature, pH, dissolved oxygen (dO₂) and the like, are those used with the particular host and will be apparent to the ordinarily skilled artisan. Generally, the pH is adjusted to a level between about 6.5 and 7.5 using either an acid (e.g., CO₂) or a base (e.g., Na2CO₃ or NaOH). A suitable temperature range for culturing mammalian cells such as CHO cells is between about 30°C to 38°C, and a suitable dO₂ is between 5-90% of air saturation.

At a particular stage the cells may be used to inoculate a production phase or step of the cell culture. Alternatively, as described above the production phase or step may be continuous with the inoculation or growth phase or step.

The cell culture environment during the production phase of the cell culture is typically controlled. Thus, if a glycoprotein is produced, factors affecting cell specific productivity of the mammalian host cell may be manipulated such that the desired sialic acid content is achieved in the resulting glycoprotein. In a preferred aspect, the production phase of the cell culture process is preceded by a transition phase of the cell culture in which parameters for the production phase of the cell culture are engaged. Further details of this process are found in U.S. Patent No. 5,721,121, and Chaderjian et al., Biotechnol. Prog. 21(2):550-3 (2005), the entire disclosures of which are expressly incorporated by reference herein.

Following culturing for a sufficient period of time, biological product, such as expressed protein, may be purified. Procedures for purification of proteins or other products from cell debris initially depend on the site of expression of the protein. Some proteins or other products can be caused to be secreted directly from the cell into the surrounding growth media; others are made intracellularly. For the latter, the first step of a purification process involves lysis of the cell, which can be done by a variety of methods, including mechanical shear, osmotic shock, or enzymatic treatments. Such disruption releases the entire contents of the cell into the homogenate, and in addition produces subcellular fragments that are difficult to remove due to their small size. These are generally removed by differential centrifugation or by filtration. The same problem arises, although on a smaller scale, with directly secreted proteins due to the natural death of cells and release of intracellular host cell proteins and components in the course of the biological product (e.g. protein) production run.

Once a clarified solution containing the biological product (e.g. protein) of interest has been obtained, its separation from the other components produced by the cell is usually attempted using a combination of different chromatography techniques. These techniques separate mixtures of products on the basis of their charge, degree of hydrophobicity, or size. Several different chromatography resins are available for each of these techniques, allowing accurate tailoring of the purification scheme to the particular product involved. The essence of each of these separation methods is that proteins or similar species can be caused either to move at different rates down a long column, achieving a physical separation that increases as they pass further down the column, or to adhere selectively to the separation medium, being then differentially eluted by different solvents. In some cases, the desired protein is separated from impurities when the impurities specifically adhere to the column, and the protein of interest does not, that is, the protein of interest is present in the "flow-through." Thus, purification of recombinant proteins from the cell culture of host cells may include one or more affinity (e.g. protein A) and/or ion exchange chomarographic steps.

In addition to mammalian host cells, other eukaryotic organisms can be used as host cells for expression of biological products, such as the recombinant protein. For expression in yeast host cells, such as common baker's yeast or *Saccharomyces cerevisiae,* suitable vectors include episomally-replicating vectors based on the 2-micron plasmid, integration vectors, and yeast artificial chromosome (YAC) vectors. Other yeast suitable for recombinant production of heterologous proteins include *Schizosaccharomyces pombe* (Beach and Nurse, Nature, 290: 140 (1981); EP 139,383 published 2 May 1985); *Kluyveromyces* hosts (U.S. Pat. No. 4,943,529; Fleer et al., Bio/Technology, 2: 968 975 (1991)) such as, *e.g., K. lactis* (MW98-8C, CBS683, CBS4574; Louvencourt et al., J. Bacteriol., 737 (1983)), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8: 135 (1990)), *K. thermotolerans,* and *K. marxianus; yarrowia* (EP 402,226); *Pichia pastoris* (EP 183,070; Sreekrishna et al., J. Basic Microbiol., 28: 265 278 (1988)); *Candida; Trichoderma reesia* (EP 244,234); *Neurospora crassa* (Case et al., Proc. Natl. Acad. Sci. USA, 76: 5259 5263 (1979*)); Schwanniomyces* such as *Schwanniomyces occidentalis* (EP 394,538 published 31 Oct. 1990); and filamentous fungi such as, *e.g., Neurospora, Penicillium, Tolypocladium* (WO 91/00357 published 10 Jan. 1991), and *Aspergillus* hosts such as A. *nidulans* (Ballance et al., Biochem. Biophys. Res. Commun., 112: 284 289 (1983); Tilburn et al., Gene, 26: 205 221 (1983); Yelton et al., Proc. Natl. Acad. Sci. USA, 81: 1470 1474 (1984)) and *A. niger* (Kelly and Hynes, EMBO J., 4: 475 479 (1985)); which are each incorporated by reference herein in their entirety. Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982), which is incorporated by reference herein in its entirety. Expression systems for the listed and other yeasts are well known in the art and/or are commercially available.

For expression in insect host cells, such as Sf9 cells, suitable vectors include baculoviral vectors. For expression in plant host cells, particularly dicotyledonous plant hosts, such as tobacco, suitable expression vectors include vectors derived from the Ti plasmid of *Agrobacterium tumefaciens.*

Suitable methods also extend to cultures of prokaryotic or archaea host cells. Prokaryotic host cells and archaea host cells suitable for expressing antibodies and other proteins to be protected by means of the instant disclosure include *Archaea* and *Eubacteria,* such as Gram-negative or Gram-positive organisms. Examples of useful bacteria include *Escherichia* (*e.g., E. coli*), *Bacilli* (*e.g., B. subtilis*), *Enterobacteria*, *Pseudomonas* species (*e.g., P. aeruginosa*), *Salmonella typhimurium, Serratia marcescans, Klebsiella, Proteus, Shigella, Rhizobia, Vitreoscilla,* or *Paracoccus.* In one embodiment, gram-negative cells are used. Examples of *E. coli* strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ(nmpc-fepE) degP41 kanR (U.S. Pat. No. 5,639,635). Other strains and derivatives thereof, such as *E. coli* 294 (ATCC 31,446), *E. coli* B, *E. coli*I 1776 (ATCC 31,537) and *E. coli* RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990), which is incorporated by reference herein in its entirety. It is generally necessary to select the appropriate bacteria taking into consideration replicability of the replicon in the cells of a bacterium. For example, *E. coli, Serratia,* or *Salmonella* species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

Methods for the production, recovery and purification of biological products such as recombinant proteins from non-mammalian host cell cultures are also well known in the art. If the polypeptide is produced in a non-mammalian cell, e.g., a microorganism such as fungi or *E. coli,* the polypeptide will be recovered inside the cell or in the periplasmic space (Kipriyanov and Little, Molecular Biotechnology, 12: 173 201 (1999); Skerra and Pluckthun, Science, 240: 1038 1040 (1988); which are each incorporated by reference herein in their entirety). Hence, it is necessary to release the protein from the cells to the extracellular medium by extraction such as cell lysis. Such disruption releases the entire contents of the cell into the homogenate, and in addition produces subcellular fragments that are difficult to remove due to their small size. These are generally removed by differential centrifugation or by filtration.

Cell lysis is typically accomplished using mechanical disruption techniques such as homogenization or head milling. While the protein of interest is generally effectively liberated, such techniques have several disadvantages (Engler, Protein Purification Process Engineering, Harrison eds., 37 55 (1994), incorporated by reference herein in its entirety). Temperature increases, which often occur during processing, may result in inactivation of the protein. Moreover, the resulting suspension contains a broad spectrum of contaminating proteins, nucleic acids, and polysaccharides. Nucleic acids and polysaccharides increase solution viscosity, potentially complicating subsequent processing by centrifugation, cross-flow filtration, or chromatography. Complex associations of these contaminants with the protein of interest can complicate the purification process and result in unacceptably low yields. Improved methods for purification of heterologous polypeptides from microbial fermentation broth or homogenate are described, for example, in U.S. Patent No. 7,169,908, the entire disclosure of which is expressly incorporated herein by reference.

In an aspect, the disclosure provides a method of culturing a cell in a medium. The method comprises preventing loss of copper in the medium according to a method of the invention, or preparing the medium according to a method of the invention; contacting the cell with the medium; and culturing the cell in the medium.

In another aspect, the disclosure provides a method of making a biological product. The method comprises preventing loss of copper in the medium according the invention or preparing a medium according to the invention; contacting a cell configured to express said biological product with the medium; and culturing the cell in the medium under conditions such that the cell expresses the biological product.

A further aspect provides use of a cell culture medium, cell culture feed, or cell culture additive of the invention for culturing cells.

It is emphasized that the culturing, fermentation, methods of making biological product, recovery and purification methods described herein are only for illustration purposes. The disclosed methods can be combined with any manufacturing process developed for the production, recovery and purification of recombinant proteins or other biological products.

### ASSAYS

### Assay for quantification of cysteine (monomer)

The concentration of cysteine may be determined using an Ellman's reagent assay for free thiols, a method commonly employed for measuring cysteine in the context of cysteine oxidation (Smith RC, et al., Oxidation Of Thiols By Copper(II). Phosphorus, Sulfur, and Silicon and the Related Elements. 1994;90(1-4):147-154; and Munday R, et al., Inhibition of copper-catalyzed cysteine oxidation by nanomolar concentrations of iron salts. Free Radical Biology and Medicine. 2004;36(6):757-764; each incorporated by reference herein in their entirety). The assay was performed by adding Ellman's reagent (DTNB, Sigma Aldrich) to the media and measuring the absorbance at 412nm. Using Beer's Law and an extinction coefficient of 13,600 M⁻¹cm⁻¹ (as per Ellman GL, Tissue sulfhydryl groups, Archives of Biochemistry and Biophysics, 1959;82(1):70-77, incorporated by reference herein in its entirety) the reading was converted to a concentration of free thiols. Provided the media does not contain any other free thiols at meaningful concentrations, this provides the concentration of cysteine monomer. If other free thiols are present, the value obtained needs to be corrected from the non-cysteine monomer thiols to provide the concentration of cysteine monomer.

### Bathocuproine assay for copper (I)

This assay may be used to confirm the presence of copper (I). A water soluble version of the chelator, bathocuproine disulfonic acid, as described in Blair D and Diehl H, Bathophenanthrolinedisulfonic acid and bathocuproinedisulfonic acid, water soluble reagents for iron and copper, Talanta. 1961;7(3-4) was used (Sigma Aldrich). Bathocuproine is a chelator that selectively binds Cu(I) (see Smith G and Wilkins DH, New Colorimetric Reagent Specific for Copper. Analytical Chemistry, 1953;25(3)) and prevents Cu(I) being oxidized to Cu(ll) by oxygen. The bathocuproine-Cu(I) complex can be quantified by its absorbance at 483 nm with an extinction coefficient of 13,300 M⁻¹ cm⁻¹, thereby determining the level of Cu(I).

### Pyruvate, acetate and trace metal determination

Pyruvate may be measured using a pyruvate assay kit, such as the pyruvate assay kit avaialable for the Cedex Bio HT instrument. The assay is enzymatic using lactate dehydrogenase and measuring the absorbance of NADH at 340 nm.

Acetate may be measured using an Ion Exchange Chromatography method. For example, acetate may be determined with a Dionex 3000 series HPLC with an RFIC lonPac AS11-HC column (Dionex) and conductivity detection. The acetate may be eluted with a KOH gradient and results for samples compared against a standard curve.

Trace metals may be quantified by the skilled person using convention methods of atomic detection, such as Inductively Coupled Plasma - Optical Emission Spectroscopy (ICP-OES) or Inductively Coupled Plasma - Mass Spectrometry (ICP-MS). In an exemplary ICP-OES method, samples may be diluted in nitric acid then aspirated into an argon plasma where the trace metals are ionized. The trace metals in the samples may be determined by spectrometric readings at specific wavelengths and intensity readings were compared to a standard curve for quantification.

### Assay for copper removal by filtration

Filtering a solution, such as a cell culture cell culture medium, cell culture feed or cell culture additive, may result in loss of insoluble copper on the filter. The amount of copper removed may be quantified after recovering the copper from the filter, for example using the "trace metal determination" assays described above. Copper may be recovered from the filter in any suitable manner.

Where the filter is made from a material that readily dissolves in specified solvent systems, the filter and any copper present on the filter may be dissolved in a suitable solvent. For example, polyethersulfone (PES) Steriflip filters may be completely dissolved with DMSO. The DMSO solution was then diluted in 5% nitric acid. The copper content of the filtered media, unfiltered media, and the dissolved filter solution were quantified by ICP-OES, thereby determining the amount of copper removed from the solution by filtering.

Another approach to recovering the copper removed from the solution is to reverse the flow to flush the removed copper from the filter. For example, for Millipore Millex PES 0.22 µm filters with a filter area of 3.9 cm², after filtering 1L of media, the filter may be flushed with a reverse flow of 50mL of 5% nitric acid by. The copper content of the filter media, unfiltered media, and the 5% nitric acid wash were quantified by ICP-OES. This method may be readily adapted to any filter that is compatible with reversing the flow.

An example of a specific assay that may be used is as follows. 1.5L of media was prepared in a 2L glass beaker and covered with parafilm to minimize oxygen transfer from the environment. The beaker was placed on a Corning PC-620D stir plate with calibrated RPM settings. The mixing was set at 60 RPM to maintain a homogenous solution without creating a vortex that would increase gas transfer. A Mettler Toledo InPro 6860i optical dissolved oxygen probe and a Mettler Toledo InPro 3253i pH/ORP (oxidation-reduction potential) sensor were incorporated. The oxygen environment was manipulated by sparging either oxygen or nitrogen through a small diameter dip tube. Copper removal was measured by pipetting 40 ml of the media into a 50ml conical tube, filtering half the volume with a steriflip, and quantifying copper removal by ICP-OES.

### EXAMPLES

### Materials and Methods

Chemically defined medium 1 (CDM1) a proprietary CHO cell culture media was used. The media is chemically-defined and protein-free containing a range of amino acids, vitamins, glucose, bicarbonate buffer, salts, and trace elements. Two of the trace elements in the media that are important to this work are iron and copper. Both are present at low µM concentrations with iron significantly more abundant than copper. Cysteine and pyruvate, two components relevant to this work, are in the low millimolar range (1-10mM) with pyruvate always in excess of cysteine. The media was titrated to a pH of 7 and had an osmolality of approximately 300mOsm.

Complete chemically defined media (CDM) recipes contain a many components. A simplified media recipe was therefore developed for studying cysteine and copper chemistry. This media maintains the pH, buffer components, ionic strength, osmolality, and trace metal ions copper and iron along with cysteine and components known to interact with cysteine, such as pyruvate.

The simplified medium as used herein was a minimal viable product version of CDM1 with all key component chemistries, pH buffering, and redox active components that were present in CDM1. Performing experiments with both CDM1 and simplified medium permitted the determination of effects with and without the full complexity of the CDM1 medium components

In order to test a large number of conditions and time points, a high throughput system was developed. This method used 50ml conical tubes and Millipore (Waltham, MA) 0.22 µM PES Steriflip filter units (unless otherwise noted). Media (CDM1 and simplified media) were prepared and 40 ml was aliquoted into conical tubes. These were then stored under ambient conditions without agitation. At various time points post-preparation, half of the volume from one aliquot was filtered using a steriflip, leaving 20ml unfiltered. The copper content of both the filtered and unfiltered media were quantified with an ICP-OES assay to determine the copper removed during filtration

### Example 1: Time course of copper removal by filtration

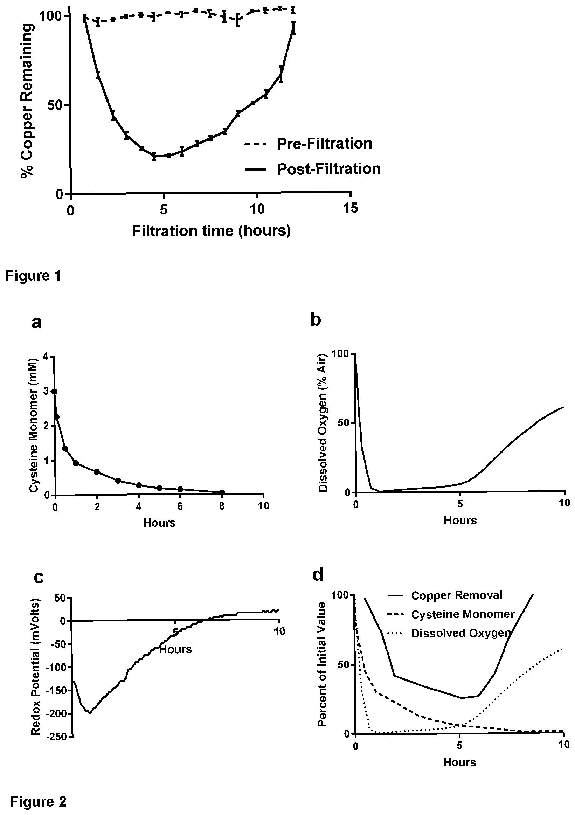

The copper species that is removed by filtration after media preparation of CDM1 media is transient. The cystine and copper concentrations in CDM1 were within the ranges for a basal medium as defined herein in "Table 1". To characterize the time dependence, we prepared media, and filtered a set of samples every 45 minutes until copper removal was no longer observed. The copper removal time course profile is shown in Figure 1. Initially copper is fully soluble and will not be removed via filtration; however after approximately 1 hour, copper begins to be removed by filtration. After 12 hours copper is once again fully filterable. Between 1 and 12 hours, copper removal displays a U-shaped curve with a maximum loss of approximately 80% occurring between 4 and 6 hours. This phenomenon is specific to copper. No loss of other measurable trace metals in the media was observed.

To confirm that the unfilterable copper is in the Cu(I) form, we used the "Bathocuproine assay for copper (I)" described above. Cysteine containing media four hours post copper addition tested positive for Cu(I), while a control case of media prepared with cystine (no copper loss observed) tested negative. Our stock solution of Cu(II)SO₄ was also negative for Cu(I).

### Example 2: Media component knockout studies

Media component knockout studies were performed. These confirmed that cysteine was the major component involved in causing copper filtration removals. When the modified media did not contain cysteine, no copper loss is observed. In media that uses cystine (the oxidized dimer form) rather than cysteine, copper removal during filtration was also not observed. We also observed that the time course transience of the copper loss was influenced by iron and pyruvate. The copper loss was pH dependent, occurring most severely around neutral pH, but was not restricted to a specific buffer species.

### Example 3: Examination of cysteine oxidation process

We monitored the cysteine oxidation process by measuring the cysteine monomer concentration, dO₂, and the redox potential over time after trace metals addition. Figure 2 shows the time courses of cysteine monomer, dO₂, redox potential, and an overlay of the cysteine monomer, dO₂, and copper loss profiles. Based on these profiles, copper loss appears to occur only while cysteine is being oxidized. These profiles are consistent between experiments.

The addition of metal ions to the media triggers a reaction that oxidizes the cysteine and consumes oxygen, causing an immediate drop in dO₂ and cysteine monomer. When the dO₂ becomes limited, we observe copper become unfilterable and the rate of decline of cysteine monomer slows considerably. The dO₂ remains low and copper remains unfilterable until cysteine monomer becomes limiting. Once the cysteine monomer is depleted, the dO₂ begins to rise (as a result of ambient diffusion) and the copper returns to its filterable form.

The reaction of cysteine monomer can also be followed by measuring the redox potential. Cysteine is a relatively strong reducing agent which causes a negative redox potential (Jocelyn PC, The Standard Redox Potential of Cysteine-Cystine from the Thiol-Disulphide Exchange Reaction with Glutathione and Lipoic Acid, European Journal of Biochemistry, 1967;2(3), incorporated by reference herein in its entirety). The redox potential profile is shown in Figure 2c. Initially as oxygen is consumed, the redox potential drops and reaches a minimum when the DO is zero. After that the redox potential becomes less reducing as cysteine monomer is consumed. As the concentration of cysteine monomer approaches zero, the redox potential stabilizes slightly above zero indicating a mild oxidizing environment, likely as a result of the cystine now in solution

The oxidation reaction of cysteine to cystine is reported to have a potential of - 220mV at pH 7 (Jocelyn PC, *ibid*), which correlates well with our observed minimum observed potential of -200 mV. Cell culture media typically contains other redox active components (e.g. tyrosine); however cysteine appears to be the most influential component during the measured time period.

In the example formulations, copper is added to the media as Cu(II)SO₄, a highly soluble form of copper (II). When catalyzing cysteine oxidation, copper is converted to and held as Cu(I), a significantly less soluble form. Without wishing to be bound by any theory, we hypothesize that a Cu(I) intermediate of cysteine oxidation, likely in complexed form with cysteine/cystine, has low solubility and forms precipitates that are removed by filtration. This is consistent with reports that many copper thiolates have very low solubility and often form precipitates.

A Pourboix (Redox potential / pH) diagram describing an aqueous system of copper, iron, and sulfur in Garrels RM, Christ CL, Eh-pH Diagrams. Harper and Row, 1965, at page 232 (which is incorporated by reference herein in its entirety) shows that, in the conditions observed in exemplary media (pH 7 and redox potential ranging from 0 to - 200 mV), there are at least 6 distinct forms that these 3 molecules may take. While we cannot simply substitute cysteine for sulfur and assume the same species arise, the diagram displays the complexity of the interactions and the transience of the species as the redox potential is shifting. It follows that during cysteine oxidation, it is reasonable o expect that there are similarly numerous copper-iron-thiol species that may occur. Without wishing to be bond by any theory, we suggest that one or more of these forms are insoluble, causing copper-containing precipitates that can be removed by filtration. While we do not observe a measurable loss of cysteine or iron, copper is typically present in cell culture media and related compositions at a concentration significantly lower than cysteine and iron. If either cysteine or iron were removed at the same molar level as copper, the losses would usually not be large enough to have a significant effect on overall the levels of cysteine and iron in the media, feed or additive composition.

### Example 4: Effect of increasing cysteine concentration

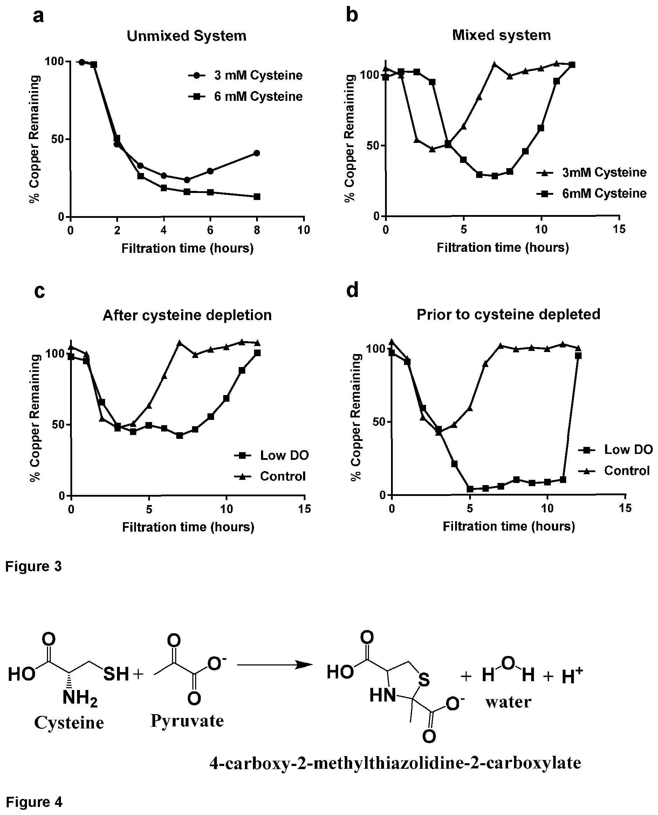

The time course of copper removal in media with two different initial cysteine concentrations, 3mM and 6mM, was investigated. Figure 3a shows the copper removal profiles with these different cysteine concentrations using a high throughput system (unmixed) and figure 3b shows the profiles in a small scale system (mixed). Increasing initial cysteine monomer extended the period of time in which copper is removed via filtration.

The low dO₂ state and time to cysteine monomer depletion were extended along with copper removal. This confirms that the reason increasing initial cysteine extended the duration of copper removal is that cysteine oxidation was occurring for longer.

The ability to control the time frame of the insoluble copper species by adjusting the cysteine monomer concentration further supports cysteine oxidation as the cause of copper removal.

### Example 5: Effect of low dO₂

Examination of Figure 2d indicated that cysteine oxidation starts as soon as metals ions are added to the media, however copper does not become unfilterable until the dO₂ becomes limiting. After the cysteine monomer becomes limiting, dO₂ starts to rise and copper becomes filterable again. It appears that low dO₂ is necessary for copper removal to occur and that oxygen is required for copper to return to a filterable form. This was confirmed by two experiments in which dO₂ was maintained at zero by sparging nitrogen into the system.

In the first experiment, nitrogen sparging was initiated at approximately 4 hours as cysteine monomer was reaching depletion and then oxygen was slowly re-introduced to the system starting at 8 hours. Figure 3c shows that copper removal was extended while nitrogen sparging maintained a zero dO₂ environment, and once the level of dO₂ started to rise, the copper became filterable again, due to being oxidized to the more soluble copper (II) oxidation state.

In the second experiment, sparging was initiated slightly before cysteine monomer was depleted (confirmed to be depleted at later time points) and was maintained until 11 hours, at which point nitrogen sparging was stopped and aggressive agitation was used to rapidly increase the dO₂. Figure 3d shows that in this experiment, after sparging was initiated the magnitude of copper removal increased to nearly 100% and stayed at that level until oxygen was re-introduced. The rapid increase in dO₂ caused an equally rapid return of copper filterability.

These two experiments show that copper removal can be extended indefinitely by maintaining the dO₂ at zero and that oxygen is required for copper to convert back to a filterable form. While the probes cannot distinguish small changes at near zero dO₂, the second experiment also shows that when nitrogen sparging is used to reach a near zero oxygen environment, copper may be quantitatively removed during filtration. This suggests that the reason we see only partial copper removal in non-nitrogen sparged experiments is that cysteine oxidation by itself is not enough to reach zero dO₂; trace levels of oxygen may remain and convert a portion of the insoluble copper (I) back to a filterable copper (II) form.

### Example 6: Reaction of cysteine with pyruvate

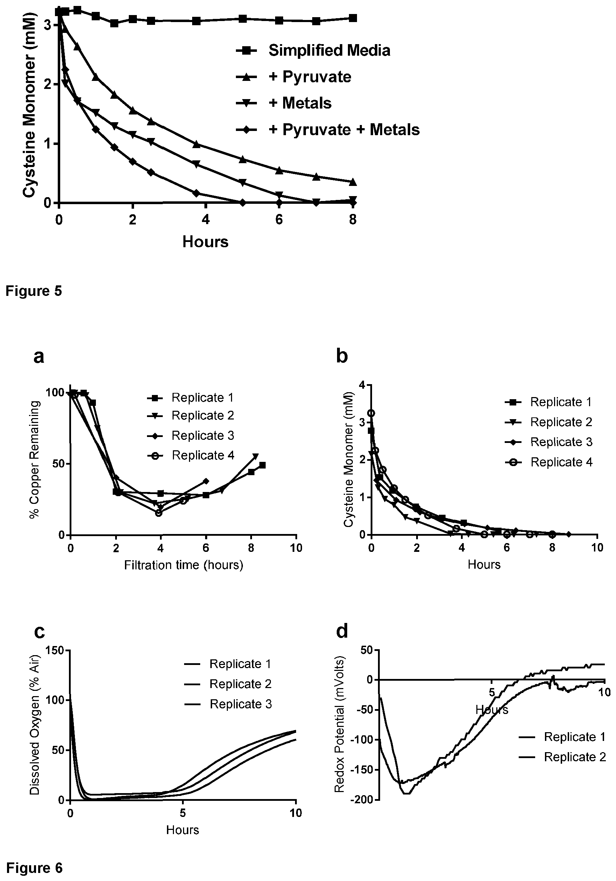

In typical cell culture media, free cysteine may take part in two main reaction pathways. The first is oxidation to cystine. The second is condensation of cysteine and pyruvate to for a 2,4 thiazolidine, as illustrated in Figure 4. These two reactions compete for cysteine monomer, however using a simplified media formulation, we modulated which reactions were occurring by removing metal ions to prevent the oxidation reaction, removing pyruvate to prevent the condensation reaction, or removing metal ions and pyruvate to prevent both reactions. We then observed the decrease in cysteine monomer concentration to gain an understanding of the relative reaction rates. Figure 5 shows the cysteine monomer profiles in simplified media +/- metal ions and +/- pyruvate.

As described in Figure 4, the condensation reaction of cysteine and pyruvate generate a molecule of water and a hydrogen ion along with the thiazolidine. When bicarbonate is used as the buffering species it is difficult to measure stoichiometric changes in pH due to outgassing of CO₂, but when we replaced bicarbonate with MOPS buffer in our simplified media we were able to measure a near stoichiometric decrease in pH for the amount of cysteine converted to thiazolidine. This shows that thiazolidine formation is responsible for the pH drop, proportional to cysteine concentration, which is observed during media holds of CDM containing cysteine and pyruvate.

Additionally, the pyruvate concentration was measured prior to cysteine addition and after cysteine monomer was depleted. In the absence of metals ions, the pyruvate concentration decreased by 3.0 mM stoichiometrically correlating with the 3.0 mM decrease in cysteine suggesting complete conversion to the thiazolidine. Additionally, in the absence of metal ions, the dO₂ does not change; offering further evidence that cysteine was not oxidized.

When metal ions were included, the pyruvate data confirmed a competition for cysteine monomer between the two reactions. The pyruvate concentration decreased less (1.7mM) suggesting partial conversion to the thiazolidine, and we calculate that the remaining cysteine (1.3mM) was oxidized to cystine (0.65mM). Additionally, the dO₂ drop characteristic of cysteine oxidation was observed.

While pyruvate is not directly involved with the mechanism of copper forming an insoluble species, the presence of pyruvate in cell culture media greatly impacts the time course of copper removal. We have shown that insoluble copper species is maintained until there is no cysteine monomer available to be oxidized, and after oxygen becomes limiting for cysteine oxidation reactions, the condensation with pyruvate is responsible for consuming much of the remaining monomer. Without pyruvate, the oxidation reaction would be the only route to cysteine consumption. Limited by oxygen availability, the oxidation reaction would take much longer to consume all of the cysteine and copper removal would have a longer duration.

When pyruvate and metal ions are present in our simplified media, we have found that a small amount of acetate (0.12 mM in our simplified media) is generated. No acetate was detected unless both pyruvate and metal ions were present. As shown in equation 1, the first step of cysteine oxidation generates hydrogen peroxide, which can go on to oxidize additional cysteine as shown by equation 2. However, hydrogen peroxide is quite reactive and likely interacts with a range of media components. One example is the oxidization of pyruvate to acetate we have observed. Equation 7 shows how this reaction is typically depicted (Nath KA,et al., alpha-Ketoacids scavenge H2O2 in vitro and in vivo and reduce menadione-induced DNA injury and cytotoxicity, American Journal of Physiology-Cell Physiology, 1995, 268(1); Giandomenico AR, et al., The importance of sodium pyruvate in assessing damage produced by hydrogen peroxide, Free Radical Biology and Medicine, 1997, 23(3), 426-434; which are each incorporated by reference herein in their entirety).

*Pyruvate + H₂O₂* → *Acetate + CO₂ + H₂O* *(7)*

The stoichiometry of cysteine oxidation described in equations 1-3 is 4:1 moles of cysteine oxidized to mole of oxygen consumed. This assumes complete reaction of hydrogen peroxide with cysteine. In media, the stoichiometry is less than 4:1 as a result of hydrogen peroxide's propensity to react with other media components such as pyruvate.

### Example 7: Effect of filter material and pore size of copper

Examples 1-6, when they used filters, used polyethersulfone (PES) membrane filters with a pore size of 0.22 µm. We have also tested polyvinylidene difluoride or polyvinylidene difluoride (PVDF) and nitrocellulose membranes and pore sizes ranging from 0.22 µm to 8 µm. We found that copper removal during filtration had no dependence on membrane material or pore size.

In addition, we also demonstrated that copper removed during filtration can be recovered from the filter material using the assays for copper removal by filtration described herein. By dissolving the filter, we were able to recover 99% of the copper removed from the media during filtration; and by simply back-flushing the filter, we were still able to recover 89% of the removed copper.
**the present application and invention further includes the subject matter of the following numbered clauses:**
1. A method of preventing loss of copper in a solution,
   wherein the solution is a cell culture medium, cell culture feed or cell culture additive comprising copper (11) and cysteine,
   wherein the method comprises having substantially the same amount of soluble copper before and after filtering the solution.
2. The method of clause 1, wherein the filtering is performed with the solution comprising a dissolved oxygen (dO₂) level of at least about 6% dO₂, optionally a dissolved oxygen (dO₂) level of at least about 8% dO₂.
3. The method of clause 2, further comprising maintaining the dO₂ level of the solution at at least about 6% dO₂ (or at least about 8%dO₂) for at least about 10 minutes prior to commencing the filtering.
4. The method of clause 2 or clause 3, wherein the dO₂; levels are maintained at the required level by:
   a. oxygenating the solution; and/or
   b. Optimizing the filtering.
5. The method of clause 4, wherein the oxygenating comprises:
   sparging the solution with a gas comprising oxygen; and/or
   agitating or mixing the solution to increase contact of the solution with gas comprising oxygen.
6. The method of clause 5, wherein the gas comprising oxygen is atmosphere.
7. The method of any of clauses 4 to 6, wherein optimizing the filtering comprises increasing filtration rate, filter size and/or filter capacity, such that dO₂ levels do not fall below 8% during filtering.
8. The method of any preceding clause, wherein the filtering is completed within about 24 hours of adding copper (II) to the solution.
9. The method of clause 1-5, wherein the filtering is performed within about 5 hours, about 1 hour, or about 30 minutes of adding copper (II) to the solution.
10. The method of any preceding clause, wherein the filtering is performed at a rate of at least 0.5% of the total volume per minute, optionally at least 5% of the total volume per minute.
11. The method of any proceeding clause, wherein the method comprises providing a cysteine solution and a copper (II) solution; and
   the filtering comprises separately filtering the cysteine solution and the copper (II) solution, and then combining the filtered cysteine solution and the filtered copper (II) solution to form the cell culture medium, cell culture feed or cell culture additive.
12. The method of clause 11, wherein the cysteine solution comprises at least the majority of the components of the cell culture medium, cell culture feed or cell culture additive, other than copper.
13. The method of clause 11 or clause 12, wherein the cysteine solution comprises substantially all of the components of the cell culture medium, cell culture feed or cell culture additive other than copper and optionally iron.
14. The method of any of clauses 11 to 13, wherein the concentration of copper in the copper (II) solution is in the range of about 0.005 µM to about 1 M.
15. The method of any preceding clause, wherein the filtering comprises sterile filtering.
16. The method of clause 15, wherein the sterile filtering comprises use of a filtration medium having a pore size of not more than about 0.5 µm, optionally not more that about 0.2 µm.
17. The method of clause 15 or clause 16, wherein the sterile filtering comprises use of a filtration medium having a pore size of at least about 0.1 µm.
18. The method of any preceding clause, wherein the cysteine is provided substantially as a cysteine derivative that does not comprise a sulfhydryl group.
19. The method of any preceding clause, wherein the cysteine is converted substantially into a cysteine derivative that does not comprise a sulfhydryl group prior to adding the copper (II) to the solution.
20. The method of clause 18 or clause 19, wherein the cysteine derivative comprises a disulfide or a thiazolidine moiety.
21. The method of any of clauses 18 to 20, wherein the cysteine derivative comprises a disulfide.
22. The method of any of clauses 18 to 21, wherein the cysteine derivative comprises a thiazolidine moiety.
23. The method of any of clauses 18 to 22, wherein the cysteine derivative is selected from a group consisting of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine).
24. The method of clause 23, wherein the cysteine derivative comprises cystine.
25. The method of clause 24, wherein the cysteine derivative comprises 4-carboxy-2- methylthiazolidine-2-carboxylate.
26. The method of any preceding clause, wherein the copper is in the form of a copper (II) chelate.
27. The method of clause 21, wherein the chelate comprises a chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2).
28. A method of preparing a cell culture medium, cell culture feed or cell culture additive comprising copper (II), cysteine, and other components, without loss of soluble copper, comprising:
   providing an aqueous solution comprising cysteine and other components;
   providing an aqueous solution comprising copper (II);
   separately filtering the aqueous solution comprising cysteine and other components, and the aqueous solution comprising copper (II); and
   combining the filtered solutions to provide the cell culture medium, cell culture feed or cell culture additive.
29. The method of clause 28, wherein preparation of the aqueous solution comprising cysteine and other components comprises dissolving and/or solubilizing cysteine and other components in water.
30. The method of clause 28 or clause 29, wherein preparation of the aqueous solution comprising copper (II) comprises dissolving a copper (II) salt in water.
31. The method of any of clauses 28 to 30, wherein the concentration of copper in the copper (II) solution is in the range of about 0.005 µM to about 1 M.
32. A method of preparing a cell culture medium, cell culture feed or cell culture additive comprising copper (II), cysteine, and other components, without loss of soluble copper, comprising:
   filtering a solution comprising the copper (II), cysteine, and other components to provide the cell culture medium, cell culture feed or cell culture additive.
33. The method of clause 32, wherein the filtering is completed within about 24 hours of adding the copper (11) to the solution;
   optionally wherein the filtering is performed within about 30 minutes of adding copper (11) to the solution.
34. The method of clause 32 or clause 33, wherein the solution is prepared by dissolving and/or solubilizing a copper (11) salt, cysteine and other components in water.
35. The method of any of clauses 28 to 34, wherein the each filtering comprises sterile filtering.
36. The method of clause 35, wherein the each sterile filtering comprises use of a filtration medium having a pore size of not more than about 0.5 µm, optionally of not more that about 0.2 µm.
37. The method of clause 35 or clause 36, wherein the sterile filtering comprises use of a filtration medium having a pore size of at least 0.1 µm.
38. The method of any of clauses 28 to 37, further comprising the features of any of clauses 2 to 7 and 18 to 27.
39. The method of any of clauses 28 to 38, wherein the other components comprise at least one carbon source, and/or at least one nitrogen source, and/or at least one vitamin, and/or at least one buffering agent, and/or at least one inorganic salt, and/or at least one trace metal other than copper, and/or at least one polyamine, and/or at least one (essential) fatty acid.
40. The method of clause 39, wherein the at least one carbon source comprises a sugar, optionally a monosaccharide or disaccharide, e.g. glucose and/or deoxyribose.
41. The method of clause 39 or 40, wherein the at least one nitrogen source comprises at least one amino acid; optionally selected from L-alanine, L-arginine, L-asparagine, L-aspartic acid, L-cystine, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine and L-valine, or derivatives thereof.
42. The method of any of clauses 39 to 41, wherein the at least one vitamin is selected from ascorbic acid, biotin, choline, D-Ca²⁺-pantothenate, folic acid, inositol, menadione, niacinamide, nicotinic acid, paraaminobenzoic acid (PABA), pyridoxal, pyridoxine, riboflavin, thiamine, vitamin A acetate, vitamin B₁₂ and vitamin D₂.
43. The method of any of clauses 39 to 42, wherein the at least one buffering agent comprises carbonate, bicarbonate, phosphate, hydrogen phosphate, dihydrogen phosphate, lactate, HEPES, MOPS, BES, TES, MES, and Bis-Tris.
44. The method of any of clauses 39 to 43, wherein the at least one inorganic salt comprises at least one of CaCl₂, KCI, MgCl₂, MgSO₄, NaCl, NaHCO₃, Na₂HPO₄,, NaH₂PO₄ and ferric citrate chelate, or ferrous sulfate chelate.
45. The method of any of clauses 39 to 44, wherein the at least one trace metal other than copper comprises ions of at least one of barium, bromium, cobalt, iodine, manganese, chromium, nickel, selenium, vanadium, titanium, germanium, molybdenum, silicon, iron, fluorine, silver, rubidium, tin, zirconium, cadmium, zinc and aluminium.
46. The method of any of clauses 39 to 45, wherein the at least one polyamine comprises at least one of putrescine, cadaverine, spermine, and spermidine.
47. The method of any of clauses 39 to 46, wherein the at least one essential fatty acid is selected from linoleic acid and linolenic acid.
48. A method of culturing a cell in a medium, the method comprising:
   preventing loss of copper in the medium according to a method of any of clauses 1 to 27, or preparing the medium according to a method of any of clauses 28 to 47,
   contacting the cell with the medium, and
   culturing the cell in the medium.
49. The method of clause 48, wherein the cell is a eukaryotic, a prokaryotic or an archaea cell.
50. The method of clauses 49, wherein the eukaryotic cell is a mammalian cell.
51. The method of clause 50, wherein the mammalian cell is selected from hybridoma cells, CHO cells, COS cells, VERO cells, Hela cells, HEK 293 cells, PER-C6 cells, K562 cells, MOLT-4 cells, MI cells, NS-1 cells, COS-7 cells, MDBK cells, MDCK cells, MRC-5 cells, WI-38 cells, WEHI cells, SP2/0 cells, BHK cells (including BHK-21 cells) and derivatives thereof.
52. The method of any of clauses 48 to 51, where the culturing the cell in the medium comprises multiplying the cell.
53. A method of making a biological product, comprising:
   preventing loss of copper in the medium according to a method of any of clauses 1 to 27 or preparing a medium according to a method of any of clauses 28 to 47,
   contacting a cell configured to express said biological product with the medium, and
   culturing the cell in the medium under conditions such that the cell expresses the biological product.
54. The method of clause 53, wherein the biological product is a polypeptide, protein, peptide, hormone, virus or virus like particle, nucleic acid or a fragment thereof; optionally an antibody, or a biologically functional fragment of an antibody.
55. The method of clause 53 or 54, further comprising isolating and/or purifying the biological product.
56. A cell culture medium, cell culture feed, or cell culture additive obtainable or obtained by the method of any of clauses 28 to 47.
57. Use of a cell culture medium, cell culture feed, or cell culture additive of clause 56 for culturing cells, optionally wherein the use comprises producing a biological product from said cells and / or optionally wherein the use comprises multiplying said cells.

## Claims

1. A method of preventing loss of copper in a solution,
wherein the solution is a cell culture medium, cell culture feed or cell culture additive comprising copper (II) and cysteine,
wherein the method comprises having substantially the same amount of soluble copper before and after filtering the solution, and
wherein the filtering is completed within about 24 hours of adding copper (II) to the solution.

2. The method of claim 1, wherein the filtering is performed within about 5 hours, about 1 hour, or about 30 minutes of adding copper (II) to the solution.

3. The method of any preceding claim, wherein the filtering is performed at a rate of at least 0.5% of the total volume per minute, optionally at least 5% of the total volume per minute.

4. The method of any proceeding claim, wherein the method comprises providing a cysteine solution and a copper (II) solution; and
the filtering comprises separately filtering the cysteine solution and the copper (II) solution, and then combining the filtered cysteine solution and the filtered copper (II) solution to form the cell culture medium, cell culture feed or cell culture additive.

5. The method of claim 4, wherein the cysteine solution comprises at least the majority of the components of the cell culture medium, cell culture feed or cell culture additive, other than copper; and/or
wherein the cysteine solution comprises substantially all of the components of the cell culture medium, cell culture feed or cell culture additive other than copper and optionally iron; and/or.
wherein the concentration of copper in the copper (II) solution is in the range of about 0.005 µM to about 1 M.

6. The method of any preceding claim, wherein the filtering comprises sterile filtering;
optionally wherein the sterile filtering comprises use of a filtration medium having a pore size of not more than about 0.5 µm, optionally not more that about 0.2 µm;
further optionally wherein the sterile filtering comprises use of a filtration medium having a pore size of at least about 0.1 µm.

7. The method of any preceding claim, wherein the cysteine is provided substantially as a cysteine derivative that does not comprise a sulfhydryl group; and/or
wherein the cysteine is converted substantially into a cysteine derivative that does not comprise a sulfhydryl group prior to adding the copper (II) to the solution;
optionally wherein the cysteine derivative comprises a disulfide or a thiazolidine moiety.

8. The method of claim 7, wherein the cysteine derivative is selected from a group consisting of cystine, 4-carboxy-2-methylthiazolidine-2-carboxylate, S-sulfocysteine, S-sulfocysteinylglycine, cysteine S-linked N-acetyl glucosamine (GlcNAC-cys), homocystine, L-cysteine mixed disulfides, L-cysteine mixed peptides, S-alkylated cysteine, cysteine with a thiol-protecting group (e.g FMOC-protected cysteine), 2-methyl-1,3-thiazolidine-2, 4-dicarboxylic acid, 2-(2-carboxyethyl)21,3-thiazolidine-2, 4-dicarboxylic acid, reduced and oxidized glutathione (GSH), S-sulfoglutathione, S-acyl-GSH, S-carboxy-L-cysteine, L-2-oxothiazolidine-4-carboxylic acid, 2-alkyl-thiazolidine-4(R)-carboxylic acid, 2-aryl-thiazolidine-4(R)-carboxylic acid and carbohydrate based thiazolidines (e.g. D-ribose-L-cysteine);
optionally wherein the cysteine derivative comprises cystine; or
optionally wherein the cysteine derivative comprises 4-carboxy-2-methylthiazolidine-2-carboxylate.

9. The method of any preceding claim, wherein the copper is in the form of a copper (II) chelate;
optionally wherein the chelate comprises a chelator selected from ethylenediaminetetraacetic acid (EDTA), diethylenetriamine pentaacetic acid (DTPA), neocuprione, bathocuprione, D-penicillamine, triethylenetetramine (TETA), dimercaprol (BAL), 8-hydroxyquinoline, clioquinol, and 5,7-Dichloro-2-[(dimethylamino)methyl]-8-quinolinol (PBT2).

10. A method of preparing a cell culture medium, cell culture feed or cell culture additive comprising copper (II), cysteine, and other components, without loss of soluble copper, comprising:
filtering a solution comprising the copper (II), cysteine, and other components to provide the cell culture medium, cell culture feed or cell culture additive; and
wherein the filtering is completed within about 24 hours of adding the copper (II) to the solution;
optionally wherein the filtering is performed within about 30 minutes of adding copper (II) to the solution.

11. The method of any of claim 10, further comprising the features of any of claims 7 to 9.

12. The method of claim 10 or 11, wherein the other components comprise at least one carbon source, and/or at least one nitrogen source, and/or at least one vitamin, and/or at least one buffering agent, and/or at least one inorganic salt, and/or at least one trace metal other than copper, and/or at least one polyamine, and/or at least one (essential) fatty acid.

13. A method of culturing a cell in a medium, the method comprising:
preventing loss of copper in the medium according to a method of any of claims 1 to 9, or preparing the medium according to a method of any of claims 10 to 12,
contacting the cell with the medium, and
culturing the cell in the medium.

14. A method of making a biological product, comprising:
preventing loss of copper in the medium according to a method of any of claims 1 to 9 or preparing a medium according to a method of any of claims 10 to 12,
contacting a cell configured to express said biological product with the medium, and
culturing the cell in the medium under conditions such that the cell expresses the biological product.

15. The method of claim 13, wherein the biological product is a polypeptide, protein, peptide, hormone, virus or virus like particle, nucleic acid or a fragment thereof; optionally an antibody, or a biologically functional fragment of an antibody; and/or.
wherein the method further comprises isolating and/or purifying the biological product.
